(19) Europäisches Patentamt
European Patent Office
Office européen des brevets

(11) EP 3 485 734 A1

(12) EUROPEAN PATENT APPLICATION

(43) Date of publication:
**22.05.2019 Bulletin 2019/21**

(51) Int Cl.:
***A21D 2/26*** *(2006.01)* ***C12N 9/24*** *(2006.01)*

(21) Application number: **17202797.1**

(22) Date of filing: **21.11.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Technische Universität München
80333 München (DE)**

(72) Inventors:
- **Graubner, Sigrid
81377 Muenchen (DE)**
- **Zverlov, Vladimir
81739 Muenchen (DE)**
- **Schwarz, Wolfgang
80637 Muenchen (DE)**
- **Hauf, Waldemar
81479 Muenchen (DE)**
- **Andreeßen, Björn
85356 Freising (DE)**
- **Bröker, Janis
85354 Freising (DE)**
- **Verheyen, Christoph
80339 Muenchen (DE)**
- **Jekle, Mario
85356 Freising (DE)**
- **Becker, Thomas
85111 Adelschlag (DE)**
- **Schulte, Philipp
80634 Muenchen (DE)**
- **Liebl, Wolfgang
85356 Freising (DE)**

(74) Representative: **Maikowski & Ninnemann
Patentanwälte Partnerschaft mbB
Postfach 15 09 20
10671 Berlin (DE)**

# (54) METHOD FOR PREPARING FOOD PRODUCTS COMPRISING RYE

(57) The present invention relates to a method for the preparation of a food product comprising rye, which comprises the steps of preparing a primary food mixture; adding to said primary food mixture a composition comprising at least one glycoside hydrolase family 10 (GH10) enzyme; and processing said primary food mixture to produce said food product comprising rye. The invention further provides GH10 enzymes, compositions comprising said enzymes and the use of said enzymes and said composition in preparing food products.

EP 3 485 734 A1

SEQUENCE LISTING

<110> Technische Universität München

<120> Method for preparing food products comprising rye

<130> MTU105EP

<160> 19

<170> PatentIn version 3.5

<210> 1
<211> 586
<212> PRT
<213> Clostridium thermocellum

<400> 1

Ala Leu Ile Tyr Asp Asp Phe Glu Thr Gly Leu Asn Gly Trp Gly Pro
1 5 10 15

Arg Gly Pro Glu Thr Val Glu Leu Thr Thr Glu Glu Ala Tyr Ser Gly
20 25 30

Arg Tyr Ser Leu Lys Val Ser Gly Arg Thr Ser Thr Trp Asn Gly Pro
35 40 45

Met Val Asp Lys Thr Asp Val Leu Thr Leu Gly Glu Ser Tyr Lys Leu
50 55 60

Gly Val Tyr Val Lys Phe Val Gly Asp Ser Tyr Ser Asn Glu Gln Arg
65 70 75 80

Phe Ser Leu Gln Leu Gln Tyr Asn Asp Gly Ala Gly Asp Val Tyr Gln
85 90 95

Asn Ile Lys Thr Ala Thr Val Tyr Lys Gly Thr Trp Thr Leu Leu Glu
100 105 110

Gly Gln Leu Thr Val Pro Ser His Ala Lys Asp Val Lys Ile Tyr Val
115 120 125

Glu Thr Glu Phe Lys Asn Ser Pro Ser Pro Gln Asp Leu Met Asp Phe
130 135 140

Tyr Ile Asp Asp Phe Thr Ala Thr Pro Ala Asn Leu Pro Glu Ile Glu
145 150 155 160

Lys Asp Ile Pro Ser Leu Lys Asp Val Phe Ala Gly Tyr Phe Lys Val
165 170 175

```
Gly Gly Ala Ala Thr Val Ala Glu Leu Ala Pro Lys Pro Ala Lys Glu
            180                 185                 190

Leu Phe Leu Lys His Tyr Asn Ser Leu Thr Phe Gly Asn Glu Leu Lys
        195                 200                 205

Pro Glu Ser Val Leu Asp Tyr Asp Ala Thr Ile Ala Tyr Met Glu Ala
    210                 215                 220

Asn Gly Gly Asp Gln Val Asn Pro Gln Ile Thr Leu Arg Ala Ala Arg
225                 230                 235                 240

Pro Leu Leu Glu Phe Ala Lys Glu His Asn Ile Pro Val Arg Gly His
                245                 250                 255

Thr Leu Val Trp His Ser Gln Thr Pro Asp Trp Phe Phe Arg Glu Asn
            260                 265                 270

Tyr Ser Gln Asp Glu Asn Ala Pro Trp Ala Ser Lys Glu Val Met Leu
        275                 280                 285

Gln Arg Leu Glu Asn Tyr Ile Lys Asn Leu Met Glu Ala Leu Ala Thr
    290                 295                 300

Glu Tyr Pro Thr Val Lys Phe Tyr Ala Trp Asp Val Val Asn Glu Ala
305                 310                 315                 320

Val Asp Pro Asn Thr Ser Asp Gly Met Arg Thr Pro Gly Ser Asn Asn
                325                 330                 335

Lys Asn Pro Gly Ser Ser Leu Trp Met Gln Thr Val Gly Arg Asp Phe
            340                 345                 350

Ile Val Lys Ala Phe Glu Tyr Ala Arg Lys Tyr Ala Pro Ala Asp Cys
        355                 360                 365

Lys Leu Phe Tyr Asn Asp Tyr Asn Glu Tyr Glu Asp Arg Lys Cys Asp
    370                 375                 380

Phe Ile Ile Glu Ile Leu Thr Glu Leu Lys Ala Lys Gly Leu Val Asp
385                 390                 395                 400

Gly Met Gly Met Gln Ser His Trp Val Met Asp Tyr Pro Ser Ile Ser
                405                 410                 415

Met Phe Glu Lys Ser Ile Arg Arg Tyr Ala Ala Leu Gly Leu Glu Ile
            420                 425                 430
```

```
Gln Leu Thr Glu Leu Asp Ile Arg Asn Pro Asp Asn Ser Gln Trp Ala
        435                 440                 445

Leu Glu Arg Gln Ala Asn Arg Tyr Lys Glu Leu Val Thr Lys Leu Val
    450                 455                 460

Asp Leu Lys Lys Glu Gly Ile Asn Ile Thr Ala Leu Val Phe Trp Gly
465                 470                 475                 480

Ile Thr Asp Ala Thr Ser Trp Leu Gly Gly Tyr Pro Leu Leu Phe Asp
                485                 490                 495

Ala Glu Tyr Lys Ala Lys Pro Ala Phe Tyr Ala Ile Val Asn Ser Val
            500                 505                 510

Pro Pro Leu Pro Thr Glu Pro Pro Val Gln Val Ile Pro Gly Asp Val
        515                 520                 525

Asn Gly Asp Gly Arg Val Asn Ser Ser Asp Leu Thr Leu Met Lys Arg
    530                 535                 540

Tyr Leu Leu Lys Ser Ile Ser Asp Phe Pro Thr Pro Glu Gly Lys Ile
545                 550                 555                 560

Ala Ala Asp Leu Asn Glu Asp Gly Lys Val Asn Ser Thr Asp Leu Leu
                565                 570                 575

Ala Leu Lys Lys Leu Val Leu Arg Glu Leu
            580                 585


<210>  2
<211>  305
<212>  PRT
<213>  Fusarium verticilloides

<400>  2

Gln Ala Ala Asp Ser Ile Asn Lys Leu Ile Lys Asn Lys Gly Lys Leu
1               5                   10                  15

Tyr Tyr Gly Thr Ile Thr Asp Pro Asn Leu Leu Gly Val Ala Lys Asp
            20                  25                  30

Thr Ala Ile Ile Lys Ala Asp Phe Gly Ala Val Thr Pro Glu Asn Ser
        35                  40                  45

Gly Lys Trp Asp Ala Thr Glu Pro Ser Gln Gly Lys Phe Asn Phe Gly
    50                  55                  60
```

```
Ser Phe Asp Gln Val Val Asn Phe Ala Gln Gln Asn Gly Leu Lys Val
65                  70                  75                  80

Arg Gly His Thr Leu Val Trp His Ser Gln Leu Pro Gln Trp Val Lys
                85                  90                  95

Asn Ile Asn Asp Lys Ala Thr Leu Thr Lys Val Ile Glu Asn His Val
            100                 105                 110

Thr Gln Val Val Gly Arg Tyr Lys Gly Lys Ile Tyr Ala Trp Asp Val
        115                 120                 125

Val Asn Glu Ile Phe Glu Trp Asp Gly Thr Leu Arg Lys Asp Ser His
    130                 135                 140

Phe Asn Asn Val Phe Gly Asn Asp Asp Tyr Val Gly Ile Ala Phe Arg
145                 150                 155                 160

Ala Ala Arg Lys Ala Asp Pro Asn Ala Lys Leu Tyr Ile Asn Asp Tyr
                165                 170                 175

Ser Leu Asp Ser Gly Ser Ala Ser Lys Val Thr Lys Gly Met Val Pro
            180                 185                 190

Ser Val Lys Lys Trp Leu Ser Gln Gly Val Pro Val Asp Gly Ile Gly
        195                 200                 205

Ser Gln Thr His Leu Asp Pro Gly Ala Ala Gly Gln Ile Gln Gly Ala
    210                 215                 220

Leu Thr Ala Leu Ala Asn Ser Gly Val Lys Glu Val Ala Ile Thr Glu
225                 230                 235                 240

Leu Asp Ile Arg Thr Ala Pro Ala Asn Asp Tyr Ala Thr Val Thr Lys
                245                 250                 255

Ala Cys Leu Asn Val Pro Lys Cys Ile Gly Ile Thr Val Trp Gly Val
            260                 265                 270

Ser Asp Lys Asn Ser Trp Arg Lys Glu His Asp Ser Leu Leu Phe Asp
        275                 280                 285

Ala Asn Tyr Asn Pro Lys Pro Ala Tyr Thr Ala Val Val Asn Ala Leu
    290                 295                 300

Arg
305
```

<210> 3
<211> 332
<212> PRT
<213> Aeromonas punctata

<400> 3

Pro Thr Glu Ile Pro Ser Leu His Ala Ala Tyr Ala Asn Thr Phe Lys
1 5 10 15

Ile Gly Ala Ala Val His Thr Arg Met Leu Gln Ser Glu Gly Glu Phe
20 25 30

Ile Ala Lys His Phe Asn Ser Ile Thr Ala Glu Asn Gln Met Lys Phe
35 40 45

Glu Glu Ile His Pro Glu Glu Asp Arg Tyr Ser Phe Glu Ala Ala Asp
50 55 60

Gln Ile Val Asp Phe Ala Val Ala Gln Gly Ile Gly Val Arg Gly His
65 70 75 80

Thr Leu Val Trp His Asn Gln Thr Ser Lys Trp Val Phe Glu Asp Thr
85 90 95

Ser Gly Ala Pro Ala Ser Arg Glu Leu Leu Leu Ser Arg Leu Lys Gln
100 105 110

His Ile Asp Thr Val Val Gly Arg Tyr Lys Gly Gln Ile Tyr Ala Trp
115 120 125

Asp Val Val Asn Glu Ala Val Glu Asp Lys Thr Asp Leu Phe Met Arg
130 135 140

Asp Thr Lys Trp Leu Glu Leu Val Gly Glu Asp Tyr Leu Leu Gln Ala
145 150 155 160

Phe Ser Met Ala His Glu Ala Asp Pro Asn Ala Leu Leu Phe Tyr Asn
165 170 175

Asp Tyr Asn Glu Thr Asp Pro Val Lys Arg Glu Lys Ile Tyr Asn Leu
180 185 190

Val Arg Ser Leu Leu Asp Lys Gly Ala Pro Val His Gly Ile Gly Leu
195 200 205

Gln Gly His Trp Asn Ile His Gly Pro Ser Ile Glu Glu Ile Arg Met
210 215 220

Ala Ile Glu Arg Tyr Ala Ser Leu Asp Val Gln Leu His Val Thr Glu
225 230 235 240

Leu Asp Met Ser Val Phe Arg His Glu Asp Arg Arg Thr Asp Leu Thr
245 250 255

Ala Pro Thr Ser Glu Met Ala Glu Leu Gln Glu Leu Arg Tyr Glu Glu
260 265 270

Ile Phe Asn Leu Phe Arg Glu Tyr Lys Ser Ser Ile Thr Ser Val Thr
275 280 285

Phe Trp Gly Val Ala Asp Asn Tyr Thr Trp Leu Asp His Phe Pro Val
290 295 300

Arg Gly Arg Lys Asn Trp Pro Phe Val Phe Asp Gln Gln Leu Gln Pro
305 310 315 320

Lys Val Ser Phe Trp Arg Ile Ile Asn Ser Met Ser
325 330

<210> 4
<211> 444
<212> PRT
<213> Artificial sequence

<220>
<223> Recombinant polypeptide

<400> 4

Asp Phe Tyr Ile Asp Asp Phe Thr Ala Thr Pro Ala Asn Leu Pro Glu
1 5 10 15

Ile Glu Lys Asp Ile Pro Ser Leu Lys Asp Val Phe Ala Gly Tyr Phe
20 25 30

Lys Val Gly Gly Ala Ala Thr Val Ala Glu Leu Ala Pro Lys Pro Ala
35 40 45

Lys Glu Leu Phe Leu Lys His Tyr Asn Ser Leu Thr Phe Gly Asn Glu
50 55 60

Leu Lys Pro Glu Ser Val Leu Asp Tyr Asp Ala Thr Ile Ala Tyr Met
65 70 75 80

Glu Ala Asn Gly Gly Asp Gln Val Asn Pro Gln Ile Thr Leu Arg Ala
85 90 95

```
Ala Arg Pro Leu Leu Glu Phe Ala Lys Glu His Asn Ile Pro Val Arg
            100                 105                 110

Gly His Thr Leu Val Trp His Ser Gln Thr Pro Asp Trp Phe Phe Arg
        115                 120                 125

Glu Asn Tyr Ser Gln Asp Glu Asn Ala Pro Trp Ala Ser Lys Glu Val
    130                 135                 140

Met Leu Gln Arg Leu Glu Asn Tyr Ile Lys Asn Leu Met Glu Ala Leu
145                 150                 155                 160

Ala Thr Glu Tyr Pro Thr Val Lys Phe Tyr Ala Trp Asp Val Val Asn
                165                 170                 175

Glu Ala Val Asp Pro Asn Thr Ser Asp Gly Met Arg Thr Pro Gly Ser
            180                 185                 190

Asn Asn Lys Asn Pro Gly Ser Ser Leu Trp Met Gln Thr Val Gly Arg
        195                 200                 205

Asp Phe Ile Val Lys Ala Phe Glu Tyr Ala Arg Lys Tyr Ala Pro Ala
    210                 215                 220

Asp Cys Lys Leu Phe Tyr Asn Asp Tyr Asn Glu Tyr Glu Asp Arg Lys
225                 230                 235                 240

Cys Asp Phe Ile Ile Glu Ile Leu Thr Glu Leu Lys Ala Lys Gly Leu
                245                 250                 255

Val Asp Gly Met Gly Met Gln Ser His Trp Val Met Asp Tyr Pro Ser
            260                 265                 270

Ile Ser Met Phe Glu Lys Ser Ile Arg Arg Tyr Ala Ala Leu Gly Leu
        275                 280                 285

Glu Ile Gln Leu Thr Glu Leu Asp Ile Arg Asn Pro Asp Asn Ser Gln
    290                 295                 300

Trp Ala Leu Glu Arg Gln Ala Asn Arg Tyr Lys Glu Leu Val Thr Lys
305                 310                 315                 320

Leu Val Asp Leu Lys Lys Glu Gly Ile Asn Ile Thr Ala Leu Val Phe
                325                 330                 335

Trp Gly Ile Thr Asp Ala Thr Ser Trp Leu Gly Gly Tyr Pro Leu Leu
            340                 345                 350
```

Phe Asp Ala Glu Tyr Lys Ala Lys Pro Ala Phe Tyr Ala Ile Val Asn
355 360 365

Ser Val Pro Pro Leu Pro Thr Glu Pro Pro Val Gln Val Ile Pro Gly
370 375 380

Asp Val Asn Gly Asp Gly Arg Val Asn Ser Ser Asp Leu Thr Leu Met
385 390 395 400

Lys Arg Tyr Leu Leu Lys Ser Ile Ser Asp Phe Pro Thr Pro Glu Gly
405 410 415

Lys Ile Ala Ala Asp Leu Asn Glu Asp Gly Lys Val Asn Ser Thr Asp
420 425 430

Leu Leu Ala Leu Lys Lys Leu Val Leu Arg Glu Leu
435 440

<210> 5
<211> 511
<212> PRT
<213> Artificial sequence

<220>
<223> Recombinant polypeptide

<400> 5

Ala Leu Ile Tyr Asp Asp Phe Glu Thr Gly Leu Asn Gly Trp Gly Pro
1 5 10 15

Arg Gly Pro Glu Thr Val Glu Leu Thr Thr Glu Glu Ala Tyr Ser Gly
20 25 30

Arg Tyr Ser Leu Lys Val Ser Gly Arg Thr Ser Thr Trp Asn Gly Pro
35 40 45

Met Val Asp Lys Thr Asp Val Leu Thr Leu Gly Glu Ser Tyr Lys Leu
50 55 60

Gly Val Tyr Val Lys Phe Val Gly Asp Ser Tyr Ser Asn Glu Gln Arg
65 70 75 80

Phe Ser Leu Gln Leu Gln Tyr Asn Asp Gly Ala Gly Asp Val Tyr Gln
85 90 95

Asn Ile Lys Thr Ala Thr Val Tyr Lys Gly Thr Trp Thr Leu Leu Glu
100 105 110

Gly Gln Leu Thr Val Pro Ser His Ala Lys Asp Val Lys Ile Tyr Val
115 120 125

Glu Thr Glu Phe Lys Asn Ser Pro Ser Pro Gln Asp Leu Met Asp Phe
130 135 140

Tyr Ile Asp Asp Phe Thr Ala Thr Pro Ala Asn Leu Pro Glu Ile Glu
145 150 155 160

Lys Asp Ile Pro Ser Leu Lys Asp Val Phe Ala Gly Tyr Phe Lys Val
165 170 175

Gly Gly Ala Ala Thr Val Ala Glu Leu Ala Pro Lys Pro Ala Lys Glu
180 185 190

Leu Phe Leu Lys His Tyr Asn Ser Leu Thr Phe Gly Asn Glu Leu Lys
195 200 205

Pro Glu Ser Val Leu Asp Tyr Asp Ala Thr Ile Ala Tyr Met Glu Ala
210 215 220

Asn Gly Gly Asp Gln Val Asn Pro Gln Ile Thr Leu Arg Ala Ala Arg
225 230 235 240

Pro Leu Leu Glu Phe Ala Lys Glu His Asn Ile Pro Val Arg Gly His
245 250 255

Thr Leu Val Trp His Ser Gln Thr Pro Asp Trp Phe Phe Arg Glu Asn
260 265 270

Tyr Ser Gln Asp Glu Asn Ala Pro Trp Ala Ser Lys Glu Val Met Leu
275 280 285

Gln Arg Leu Glu Asn Tyr Ile Lys Asn Leu Met Glu Ala Leu Ala Thr
290 295 300

Glu Tyr Pro Thr Val Lys Phe Tyr Ala Trp Asp Val Val Asn Glu Ala
305 310 315 320

Val Asp Pro Asn Thr Ser Asp Gly Met Arg Thr Pro Gly Ser Asn Asn
325 330 335

Lys Asn Pro Gly Ser Ser Leu Trp Met Gln Thr Val Gly Arg Asp Phe
340 345 350

Ile Val Lys Ala Phe Glu Tyr Ala Arg Lys Tyr Ala Pro Ala Asp Cys
355 360 365

Lys Leu Phe Tyr Asn Asp Tyr Asn Glu Tyr Glu Asp Arg Lys Cys Asp
    370                 375                 380

Phe Ile Ile Glu Ile Leu Thr Glu Leu Lys Ala Lys Gly Leu Val Asp
385                 390                 395                 400

Gly Met Gly Met Gln Ser His Trp Val Met Asp Tyr Pro Ser Ile Ser
                405                 410                 415

Met Phe Glu Lys Ser Ile Arg Arg Tyr Ala Ala Leu Gly Leu Glu Ile
            420                 425                 430

Gln Leu Thr Glu Leu Asp Ile Arg Asn Pro Asp Asn Ser Gln Trp Ala
        435                 440                 445

Leu Glu Arg Gln Ala Asn Arg Tyr Lys Glu Leu Val Thr Lys Leu Val
    450                 455                 460

Asp Leu Lys Lys Glu Gly Ile Asn Ile Thr Ala Leu Val Phe Trp Gly
465                 470                 475                 480

Ile Thr Asp Ala Thr Ser Trp Leu Gly Gly Tyr Pro Leu Leu Phe Asp
                485                 490                 495

Ala Glu Tyr Lys Ala Lys Pro Ala Phe Tyr Ala Ile Val Asn Ser
            500                 505                 510

<210> 6
<211> 369
<212> PRT
<213> Artificial sequence

<220>
<223> Recombinant polypeptide

<400> 6

Asp Phe Tyr Ile Asp Asp Phe Thr Ala Thr Pro Ala Asn Leu Pro Glu
1               5                   10                  15

Ile Glu Lys Asp Ile Pro Ser Leu Lys Asp Val Phe Ala Gly Tyr Phe
            20                  25                  30

Lys Val Gly Gly Ala Ala Thr Val Ala Glu Leu Ala Pro Lys Pro Ala
        35                  40                  45

Lys Glu Leu Phe Leu Lys His Tyr Asn Ser Leu Thr Phe Gly Asn Glu
    50                  55                  60

```
Leu Lys Pro Glu Ser Val Leu Asp Tyr Asp Ala Thr Ile Ala Tyr Met
65                  70                  75                  80

Glu Ala Asn Gly Gly Asp Gln Val Asn Pro Gln Ile Thr Leu Arg Ala
                85                  90                  95

Ala Arg Pro Leu Leu Glu Phe Ala Lys Glu His Asn Ile Pro Val Arg
            100                 105                 110

Gly His Thr Leu Val Trp His Ser Gln Thr Pro Asp Trp Phe Phe Arg
        115                 120                 125

Glu Asn Tyr Ser Gln Asp Glu Asn Ala Pro Trp Ala Ser Lys Glu Val
    130                 135                 140

Met Leu Gln Arg Leu Glu Asn Tyr Ile Lys Asn Leu Met Glu Ala Leu
145                 150                 155                 160

Ala Thr Glu Tyr Pro Thr Val Lys Phe Tyr Ala Trp Asp Val Val Asn
                165                 170                 175

Glu Ala Val Asp Pro Asn Thr Ser Asp Gly Met Arg Thr Pro Gly Ser
            180                 185                 190

Asn Asn Lys Asn Pro Gly Ser Ser Leu Trp Met Gln Thr Val Gly Arg
        195                 200                 205

Asp Phe Ile Val Lys Ala Phe Glu Tyr Ala Arg Lys Tyr Ala Pro Ala
    210                 215                 220

Asp Cys Lys Leu Phe Tyr Asn Asp Tyr Asn Glu Tyr Glu Asp Arg Lys
225                 230                 235                 240

Cys Asp Phe Ile Ile Glu Ile Leu Thr Glu Leu Lys Ala Lys Gly Leu
                245                 250                 255

Val Asp Gly Met Gly Met Gln Ser His Trp Val Met Asp Tyr Pro Ser
            260                 265                 270

Ile Ser Met Phe Glu Lys Ser Ile Arg Arg Tyr Ala Ala Leu Gly Leu
        275                 280                 285

Glu Ile Gln Leu Thr Glu Leu Asp Ile Arg Asn Pro Asp Asn Ser Gln
    290                 295                 300

Trp Ala Leu Glu Arg Gln Ala Asn Arg Tyr Lys Glu Leu Val Thr Lys
305                 310                 315                 320
```

```
Leu Val Asp Leu Lys Lys Glu Gly Ile Asn Ile Thr Ala Leu Val Phe
                325                 330                 335

Trp Gly Ile Thr Asp Ala Thr Ser Trp Leu Gly Gly Tyr Pro Leu Leu
            340                 345                 350

Phe Asp Ala Glu Tyr Lys Ala Lys Pro Ala Phe Tyr Ala Ile Val Asn
        355                 360                 365

Ser


<210>  7
<211>  201
<212>  PRT
<213>  Herbivorax saccincola

<400>  7

Arg Thr Val Thr Ser Asn Glu Ile Gly Thr His Gly Gly Tyr Asp Phe
1               5                   10                  15

Glu Phe Trp Val Asp Ser Gly Ser Gly Ser Met Thr Leu Lys Asp Gly
            20                  25                  30

Gly Ala Phe Ser Cys Gln Trp Ser Asn Ile Asn Asn Ile Leu Phe Arg
        35                  40                  45

Lys Gly Arg Lys Phe Asp Gln Thr Lys Thr His Gln Gln Leu Gly Asn
    50                  55                  60

Ile Val Val Glu Tyr Ala Ala Asp Tyr Arg Pro Asn Gly Asn Ser Tyr
65                  70                  75                  80

Leu Cys Ile Tyr Gly Trp Thr Val Asp Pro Leu Val Glu Tyr Tyr Ile
                85                  90                  95

Ile Glu Ser Trp Gly Asn Trp Arg Pro Pro Gly Ala Gln Ser Lys Gly
            100                 105                 110

Met Ile Thr Val Asp Gly Gly Thr Tyr Asp Ile Tyr Glu Thr Thr Arg
        115                 120                 125

Val Asn Gln Pro Ser Ile Ile Gly Thr Ala Thr Phe Gln Gln Tyr Trp
    130                 135                 140

Ser Val Arg Thr Ser Lys Lys Thr Ser Gly Thr Val Ser Val Ser Gln
145                 150                 155                 160
```

His Phe Arg Ala Trp Glu Ser Met Gly Met Lys Met Gly Lys Met Tyr
165 170 175

Glu Val Ala Thr Thr Val Glu Gly Tyr Gln Ser Ser Gly Ser Ala Asp
180 185 190

Val Tyr Lys Asn Val Ile Thr Ile Gly
195 200

<210> 8
<211> 44
<212> DNA
<213> Artificial sequence

<220>
<223> Synthetic oligonucleotide

<400> 8
ttaagaagga gatatacata tggctctgat ttacgatgat tttg 44

<210> 9
<211> 49
<212> DNA
<213> Artificial sequence

<220>
<223> Synthetic oligonucleotide

<400> 9
tcagtggtgg tggtggtggt gctcgagaag ttctctcaga acgagtttt 49

<210> 10
<211> 43
<212> DNA
<213> Artificial sequence

<220>
<223> Synthetic oligonucleotide

<400> 10
ttaagaagga gatatacata tggatttcta tattgacgat ttc 43

<210> 11
<211> 42
<212> DNA
<213> Artificial sequence

<220>
<223> Synthetic oligonucleotide

<400> 11
tcagtggtgg tggtggtggt ggctgttaac tatagcataa aa 42

<210> 12

<211> 47
<212> DNA
<213> Artificial sequence

<220>
<223> Synthetic oligonucleotide

<400> 12
ttaagaagga gatatacata tgcgtactgt aacatcaaat gaaatag 47

<210> 13
<211> 54
<212> DNA
<213> Artificial sequence

<220>
<223> Synthetic oligonucleotide

<400> 13
tcagtggtgg tggtggtggt gctcgaggcc aatggtaatt acgtttttat aaac 54

<210> 14
<211> 1758
<212> DNA
<213> Clostridium thermocellum

<400> 14
gctctgattt acgatgattt tgaaacaggt ctgaacggat ggggaccaag aggaccggaa 60

accgtcgaac ttaccaccga ggaagcttac tcgggaagat acagtttgaa ggtcagcgga 120

cgtaccagca catggaacgg gcccatggtt gacaaaaccg atgtgttgac tttgggcgaa 180

agctataagt tgggcgtata tgtaaaattc gtgggtgatt cctattcaaa tgagcaaaga 240

ttcagtttgc agcttcaata taacgacgga gcaggagatg tataccaaaa tataaaaacc 300

gccacggttt acaagggaac atggactttg ctggaaggac agcttacagt tcccagccat 360

gcaaaggacg taaaaatata tgtggaaacc gaatttaaaa attctccgag tccgcaggac 420

ttgatggatt tctatattga cgatttcaca gcaacacctg caaatttgcc tgaaattgag 480

aaagatattc caagcttgaa agatgtcttt gccggttatt tcaaagtggg tggtgccgca 540

actgtggcgg aactggcgcc gaagcctgca aaagagcttt tcctcaagca ttataacagc 600

ttgacttttg gtaatgagtt aaaaccggaa agtgtacttg actatgatgc tacaattgct 660

tatatggagg caaacggagg cgaccaggtt aatccgcaga taaccttgag agcggcaaga 720

cccctgttgg agtttgcgaa agaacacaac atacctgtaa gaggacatac ccttgtatgg 780

cacagccaga caccggactg gttcttcaga gaaaattact ctcaggacga aaatgctccc 840

tgggcatcca aggaagtaat gctgcaaagg ttggaaaact acataaagaa tttaatggaa 900

gctttggcga ccgaatatcc gacggttaag ttctatgcat gggacgttgt gaatgaggct 960

gttgatccta atacttcaga cggtatgaga actccgggtt cgaataacaa aaatcccgga 1020

agctccctgt ggatgcaaac cgttggaaga gattttattg ttaaagcttt tgaatatgca 1080

agaaaatatg ctcctgcgga ttgtaaactc ttctacaatg actataatga atatgaagac 1140

agaaaatgtg attttattat tgaaattctt accgaactta aagccaaagg cctggttgac 1200

ggtatgggta tgcaatccca ctgggttatg gattatccaa gcataagcat gtttgaaaaa 1260

tccatcagaa gatatgcagc attgggattg gaaattcagc ttaccgagct ggatataaga 1320

aatcctgaca acagccagtg ggctttggaa cgtcaggcta atcgttataa ggagcttgta 1380

acaaaattgg tcgatttgaa aaaagaaggc ataaacatta cggcattggt attctgggga 1440

ataaccgacg cgacaagctg gcttggagga tatccgctcc tgtttgacgc ggaatacaag 1500

gcaaaacctg cattttatgc tatagttaac agcgttccgc cgcttccgac agaaccgccg 1560

gttcaggtta tacccggtga tgtaaacggt gacggtcgtg taaattcatc cgacttgact 1620

cttatgaaaa gataccttttt aaaatccata agcgacttcc cgacaccgga aggaaaaatt 1680

gcggcggatt taaacgaaga cggcaaggta aactcgacag atttgttagc gctgaaaaaa 1740

ctcgttctga gagaactt 1758

<210> 15
<211> 915
<212> DNA
<213> Fusarium verticilloides

<400> 15
caagctgcag actccataaa caaactaatc aagaataaag gcaagcttta ctacggaact 60

attactgatc ctaatctgct tggtgtcgcc aaggataccg ccatcattaa agcagatttt 120

ggtgccgtca ctcccgagaa cagtggcaag tgggatgcaa cggaaccatc tcagggtaag 180

tttaactttg gttctttcga tcaagtcgta aattttgcac agcagaatgg attgaaagtt 240

cgtggacata ctcttgtctg gcacagtcaa ttgccacagt gggtgaagaa cattaatgac 300

aaggctaccc taacgaaagt tatcgagaat cacgttactc aggttgtagg cagatataaa 360

ggaaaaattt acgcatggga tgtagtaaat gaaatttttg agtgggatgg tacattgcgt 420

aaggattcac atttcaacaa cgtattcggc aacgatgact atgtgggaat agcttttcgt 480

gcagctcgta aggctgatcc caacgcaaag ctttacatta acgactactc tttggattca 540

ggctccgcta gtaaggtcac aaagggtatg gttccctccg tgaaaaaatg gcttagtcaa 600

ggagtacccg tggatggtat aggaagtcaa acccacctag accctggagc agcaggccag 660

atccaaggag ccctaacagc acttgctaat agtggcgtta aagaagttgc catcacagaa 720

cttgatatca ggactgcccc cgcaaatgac tacgcaacag ttacaaaagc ctgtctaaat 780

gtccctaagt gtatcggaat cacggtctgg ggcgtctctg acaagaacag ttggcgtaag 840

gagcacgact cactactatt tgatgctaac tataatccca aacccgcata taccgctgtg 900

gtaaatgccc ttcgt 915

<210> 16
<211> 1332
<212> DNA
<213> Artificial sequence

<220>
<223> Recombinant polynucleotide

<400> 16
gatttctata ttgacgattt cacagcaaca cctgcaaatt tgcctgaaat tgagaaagat 60

attccaagct tgaaagatgt ctttgccggt tatttcaaag tgggtggtgc cgcaactgtg 120

gcggaactgg cgccgaagcc tgcaaaagag cttttcctca agcattataa cagcttgact 180

tttggtaatg agttaaaacc ggaaagtgta cttgactatg atgctacaat tgcttatatg 240

gaggcaaacg gaggcgacca ggttaatccg cagataacct tgagagcggc aagacccctg 300

ttggagtttg cgaaagaaca caacatacct gtaagaggac atacccttgt atggcacagc 360

cagacaccgg actggttctt cagagaaaat tactctcagg acgaaaatgc tccctgggca 420

tccaaggaag taatgctgca aaggttggaa aactacataa agaatttaat ggaagctttg 480

gcgaccgaat atccgacggt taagttctat gcatgggacg ttgtgaatga ggctgttgat 540

cctaatactt cagacggtat gagaactccg ggttcgaata acaaaaatcc cggaagctcc 600

ctgtggatgc aaaccgttgg aagagatttt attgttaaag cttttgaata tgcaagaaaa 660

tatgctcctg cggattgtaa actcttctac aatgactata atgaatatga agacagaaaa 720

tgtgatttta ttattgaaat tcttaccgaa cttaaagcca aaggcctggt tgacggtatg 780

ggtatgcaat cccactgggt tatggattat ccaagcataa gcatgtttga aaaatccatc 840

agaagatatg cagcattggg attggaaatt cagcttaccg agctggatat aagaaatcct 900

gacaacagcc agtgggcttt ggaacgtcag gctaatcgtt ataaggagct tgtaacaaaa 960

ttggtcgatt tgaaaaaaga aggcataaac attacggcat tggtattctg ggggaataacc 1020

gacgcgacaa gctggcttgg aggatatccg ctcctgtttg acgcggaata caaggcaaaa 1080

cctgcatttt atgctatagt taacagcgtt ccgccgcttc cgacagaacc gccggttcag 1140

gttatacccg gtgatgtaaa cggtgacggt cgtgtaaatt catccgactt gactcttatg 1200

aaaagatacc ttttaaaatc cataagcgac ttcccgacac cggaaggaaa aattgcggcg 1260

gatttaaacg aagacggcaa ggtaaactcg acagatttgt tagcgctgaa aaaactcgtt 1320

ctgagagaac tt 1332

<210> 17
<211> 1533
<212> DNA
<213> Artificial sequence

<220>
<223> Recombinant polynucleotide

<400> 17

```
gctctgattt acgatgattt tgaaacaggt ctgaacggat ggggaccaag aggaccggaa      60
accgtcgaac ttaccaccga ggaagcttac tcgggaagat acagtttgaa ggtcagcgga     120
cgtaccagca catggaacgg gcccatggtt gacaaaaccg atgtgttgac tttgggcgaa     180
agctataagt tgggcgtata tgtaaaattc gtgggtgatt cctattcaaa tgagcaaaga     240
ttcagtttgc agcttcaata taacgacgga gcaggagatg tataccaaaa tataaaaacc     300
gccacggttt acaagggaac atggactttg ctggaaggac agcttacagt tcccagccat     360
gcaaaggacg taaaaatata tgtggaaacc gaatttaaaa attctccgag tccgcaggac     420
ttgatggatt tctatattga cgatttcaca gcaacacctg caaatttgcc tgaaattgag     480
aaagatattc caagcttgaa agatgtcttt gccggttatt tcaaagtggg tggtgccgca     540
actgtggcgg aactggcgcc gaagcctgca aaagagcttt tcctcaagca ttataacagc     600
ttgacttttg gtaatgagtt aaaaccggaa agtgtacttg actatgatgc tacaattgct     660
tatatggagg caaacggagg cgaccaggtt aatccgcaga taaccttgag agcggcaaga     720
cccctgttgg agtttgcgaa agaacacaac atacctgtaa gaggacatac ccttgtatgg     780
cacagccaga caccggactg gttcttcaga gaaaattact ctcaggacga aaatgctccc     840
tgggcatcca aggaagtaat gctgcaaagg ttggaaaact acataaagaa tttaatggaa     900
gctttggcga ccgaatatcc gacggttaag ttctatgcat gggacgttgt gaatgaggct     960
gttgatccta atacttcaga cggtatgaga actccgggtt cgaataacaa aaatcccgga    1020
agctccctgt ggatgcaaac cgttggaaga gattttattg ttaaagcttt tgaatatgca    1080
agaaaatatg ctcctgcgga ttgtaaactc ttctacaatg actataatga atatgaagac    1140
agaaaatgtg attttattat tgaaattctt accgaactta aagccaaagg cctggttgac    1200
ggtatgggta tgcaatccca ctgggttatg gattatccaa gcataagcat gtttgaaaaa    1260
tccatcagaa gatatgcagc attgggattg gaaattcagc ttaccgagct ggatataaga    1320
aatcctgaca acagccagtg ggctttggaa cgtcaggcta atcgttataa ggagcttgta    1380
acaaaattgg tcgatttgaa aaaagaaggc ataaacatta cggcattggt attctgggga    1440
ataaccgacg cgacaagctg gcttggagga tatccgctcc tgtttgacgc ggaatacaag    1500
gcaaaacctg cattttatgc tatagttaac agc                                 1533
```

<210> 18
<211> 1107
<212> DNA
<213> Artificial sequence

<220>
<223> Recombinant polynucleotide

<400> 18
gatttctata ttgacgattt cacagcaaca cctgcaaatt tgcctgaaat tgagaaagat 60

attccaagct tgaaagatgt ctttgccggt tatttcaaag tgggtggtgc cgcaactgtg 120

gcggaactgg cgccgaagcc tgcaaaagag cttttcctca agcattataa cagcttgact 180

tttggtaatg agttaaaacc ggaaagtgta cttgactatg atgctacaat tgcttatatg 240

gaggcaaacg gaggcgacca ggttaatccg cagataacct tgagagcggc aagacccctg 300

ttggagtttg cgaaagaaca caacatacct gtaagaggac atacccttgt atggcacagc 360

cagacaccgg actggttctt cagagaaaat tactctcagg acgaaaatgc tccctgggca 420

tccaaggaag taatgctgca aaggttggaa aactacataa agaatttaat ggaagctttg 480

gcgaccgaat atccgacggt taagttctat gcatgggacg ttgtgaatga ggctgttgat 540

cctaatactt cagacggtat gagaactccg ggttcgaata acaaaaatcc cggaagctcc 600

ctgtggatgc aaaccgttgg aagagatttt attgttaaag cttttgaata tgcaagaaaa 660

tatgctcctg cggattgtaa actcttctac aatgactata atgaatatga agacagaaaa 720

tgtgatttta ttattgaaat tcttaccgaa cttaaagcca aaggcctggt tgacggtatg 780

ggtatgcaat cccactgggt tatggattat ccaagcataa gcatgtttga aaaatccatc 840

agaagatatg cagcattggg attggaaatt cagcttaccg agctggatat aagaaatcct 900

gacaacagcc agtgggcttt ggaacgtcag gctaatcgtt ataaggagct tgtaacaaaa 960

ttggtcgatt tgaaaaaaga aggcataaac attacggcat tggtattctg ggaataacc 1020

gacgcgacaa gctggcttgg aggatatccg ctcctgtttg acgcggaata caaggcaaaa 1080

cctgcatttt atgctatagt taacagc 1107

<210> 19
<211> 603
<212> DNA
<213> Herbivorax saccincola

<400> 19
cgtactgtaa catcaaatga aataggcaca cacgggggat atgactttga attttgggta 60

gattccggtt cagggtctat gactttaaaa gacggcgggg cttttagctg tcagtggagt 120

aatataaaca atatattatt ccgtaaaggc cgcaaatttg accaaaccaa gacacatcaa 180

caacttggta atattgtggt ggaatacgca gctgactacc gtccaaatgg aaactcatac 240

ctatgtatct acggttggac agttgatccc ctggtagagt actatatcat tgaaagctgg 300

ggcaactggc gtcctccggg agcacagtca aagggtatga ttacagtgga cggcggtaca 360

tacgacattt atgagactac aagggttaac cagccttcca ttataggtac tgcaactttc 420

```
caacagtatt ggagtgttag aacatctaaa aaaacaagcg gtactgtatc tgtaagccag      480
cacttcagag cttgggaaag catgggcatg aaaatgggta aaatgtatga agttgctact      540
acagtagagg gataccagag cagcggttct gcagacgttt ataaaaacgt aattaccatt      600
ggc                                                                    603
```

## Description

### Field of the invention

The present invention relates to a method for the preparation of food products comprising rye, wherein said method comprises the steps of preparing a primary food mixture; adding to said primary food mixture a composition comprising at least one glycoside hydrolase family 10 (GH10) enzyme; and processing said primary food mixture to produce said food products comprising rye. The invention further provides GH10 enzymes, compositions comprising said enzymes and the use of said enzymes and said compositions in preparing food products.

### Background of the invention

Food products comprising rye are widely used in the human diet due to dietary benefits for human health. Such benefits are based on favorable non-starch polysaccharide content such as a high arabinoxylan (AX) content. However, processing of primary food mixtures comprising rye and producing final food products by e.g. baking is hampered by the inability of rye flour to form a viscoelastic protein network that can retain gas, such as found in the gluten network of wheat dough (Courtin & Delcour, 2002). This results in a compact, wet and sticky crumb structure as well as in bakery products with compact crumb and reduced loaf volume. Consequently, the high AX content is in equal measure boon and bane of food products comprising rye. For rye dough structure, it is hypothesized that the presence of water unextractable arabinoxylan (WU-AX) prevents the formation of a coherent protein-network due to its strong water-holding capacity, while water-extractable AX (WE-AX) and solubilized AX (S-AX) with a high molecular weight have a positive effect by forming highly viscous aqueous solutions, stabilizing the dough foam structure (Izydorczyk & Biliaderis, 1995; Meuser & Suckow, 1986; Courtin & Roelants, 1999).

One possible approach for improving protein aggregation in rye dough is to remove or reduce the WU-AX layers around protein particles by employing enzymes with xylanase activity. Depending on their specificity, xylanases cleave the glycosidic bond of the linear (1→4)-beta-D-xylopyranose backbone of AX. The majority of xylanases can be found in the glycoside hydrolase (GH) families 5, 7, 8, 10, 11 and 43 (Lombard et al., 2013); however, according to the Carbohydrate-Active enZYmes Database (CAZy) they also appear in other families such as 16, 51, 52, 62 (Adelsberger et al., 2004; Bouraoui et al., 2016; Collins et al., 2005). The most prominent enzyme families for xylanases are GH10 and GH11, which differ significantly in both, their physicochemical properties and their substrate specificity.

Despite sometimes low sequence similarity between two members of GH10 enzymes (often below 30 % of the amino acid (aa) residues in the catalytic module), the three-dimensional secondary structure of family 10 xylanases is characterized by a $(beta/alpha)_8$ barrel structure which forms a bowl shape (Larson et al., 2003), whereas GH11 enzymes show a beta-jelly role type of folding. This three-dimensional structure of the enzymes can be predicted from the sequence and proteins can have an identical fold even if there is very low sequence identity. The active site and the substrate binding amino acid residues are relatively well conserved even though these conserved aa residues are rather short sequences in respect to the complete protein sequence.

Family 10 xylanases generally have a higher molecular weight and lower pi compared with family 11 xylanases (Kolenová et al. 2006; Collins et al. 2005, Biely et al., 1997). GH10 xylanases can cleave the xylan backbone much closer to backbone decorations such as 1,2- or 1,3-alpha-L-arabinofuranosidic and 1,2-*O*-methyl-alpha-D-glucuronic acid side groups than GH11 xylanases (Biely et al., 2016).

Xylanases commonly used to increase the volume of wheat containing bakery products are mainly comprised in GH family 11. However, they fail to increase the quality of bakery products made from rye; especially, they fail to improve characteristics such as crumb structure and loaf volume (Döring et al., 2017).

### Summary of the invention

It was therefore the objective of the invention to provide a method for the preparation of food products comprising rye, which overcomes the obstacles of the prior art.

This objective is solved by a method according to claim 1, said method comprising the step of adding to a food product comprising rye a composition comprising at least one GH family 10 (GH10) enzyme. It was surprisingly found that adding family GH10 xylanases to food products comprising rye during processing of primary food mixtures such as doughs for baking significantly improved dough processing and the quality of the bakery products after baking. Food products comprising rye showed a less compact dough structure and increased softness when treated with GH10 xylanases during their preparation. This led to a significant volume increase and a softer crumb structure. According to their type of hydrolytic activity, GH10 xylanases reduce the molecular weight of AX, leading to a more favorable WU-AX to WE-AX ratio and thereby to improved food products.

In one embodiment, said at least one GH10 enzyme is comprised in the primary food mixture or used to prepare the food product in a form selected from the group consisting of a cell extract, a cell-free extract, a partially purified protein and a purified protein.

In one embodiment, said at least one GH10 enzyme is isolated from a microorganism.

In one embodiment, said at least one GH10 enzyme is a recombinant enzyme.

In one embodiment, said at least one GH10 enzyme comprises, essentially consists of or consists of a polypeptide which has at least 75 % amino acid sequence identity to a polypeptide selected from SEQ ID NO: 1 to 6 and which shows hemicellulolytic activity.

The invention further provides a GH10 enzyme with hemicellulolytic activity, which comprises, essentially consists of or consists of a polypeptide which has at least 75 % amino acid sequence identity to the polypeptide according to SEQ ID NO 4 and 5 or the polypeptide according to SEQ ID NO: 6.

In one embodiment the GH10 enzyme with hemicellulolytic activity comprises, essentially consists of or consists of a polypeptide having at least 75 % amino acid sequence identity to a polypeptide of SEQ ID NO: 4, 5, or 6.

The invention further relates to a nucleic acid molecule comprising a nucleic acid sequence encoding the GH10 enzyme with hemicellulolytic activity according to the invention.

The invention also relates to a host cell expressing the GH10 enzyme with hemicellulolytic activity according to the invention. In one embodiment, said host cell is *E. coli* or a *Bacillus* cell. Furthermore, the invention provides a method of producing GH10 enzyme with hemicellulolytic activity, the method comprising culturing said host cell under conditions permitting the production of the enzyme, and recovering the enzyme from the culture.

In one embodiment, the invention provides a composition comprising at least one GH10.

The invention further relates to methods of using at least one GH10 enzyme for producing food products.

**Detailed description of the invention**

In a preferred embodiment, the invention provides a method for the preparation of a food product comprising rye, said rye comprising arabinoxlyan, and said method comprising the step of adding to said food product a composition comprising at least one GH10 enzyme.

More preferably, said at least one GH10 enzyme has hemicellulolytic activity, most preferably xylanolytic activity.

"Hemicellulolytic activity" according to the invention is defined as the capability of an enzyme to hydrolyze hemicellulose. Hemicellulose is a common name for polysaccharides that can be prepared by alkaline extraction of plant tissues. Some of the main polysaccharides that constitute hemicellulose are xylan, glucuronoxylan, arabinoxylan, glucomannan, mixed-linkage beta-glucan and xyloglucan. Enzymes that depolymerize these polysaccharides by hydrolytic activity are called hemicellulases. Xylanases are one representative class of enzymes that belong to the hemicellulase group.

"Xylanase activity" according to the invention is defined as the capability of an enzyme to degrade the linear polysaccharide beta-1,4-xylan backbone of xylan into shorter oligosaccharides. In particular, according to the invention, xylanase activity means altering the polymeric arabinoxylan content in a cereal source to overcome the limitations of products comprising rye e.g. compact, wet and sticky crumb structure as well as of bakery products with compact crumb and reduced loaf volume. Altering the polymeric arabinoxylan content means in one embodiment the reduction of the content of polymeric arabinoxylan. In another embodiment, altering the polymeric arabinoxylan content means changing the ratio from insoluble (WU-AX) to soluble (WE-AX) arabinoxylan.

"GH10 enzyme", "GH10 xylanase" or "family 10 xylanase" according to the invention means enzymes, which have a 3-dimensional secondary structure, which is characterized by a $(beta/alpha)_8$ barrel structure which forms a bowl shape (Larson et al., 2003). In contrast, GH11 enzymes show a beta-jelly role type of folding. The active site and the substrate binding amino acid residues are relatively well conserved even though these conserved amino acid residues are rather short sequences in respect to the complete protein sequence. Family 10 xylanases generally have a higher molecular weight and lower pi compared with family 11 (Kolenová et al. 2006; Collins et al. 2005, Biely et al., 1997). GH10 xylanases can cleave the xylan backbone much closer to backbone decorations such as 1,2- or 1,3-alpha-L-arabinofuranosidic and 1,2-4-O-methyl-alpha-D-glucuronic acid side groups than GH11 xylanases (Biely et al., 2016). The classification of enzymes into the GH family follows the criteria disclosed on the Carbohydrate-Active enZYmes Database (CAZy, http://www.cazy.org/Glycoside-Hydrolases.html): There is a direct relationship between sequence and folding similarities, and such a classification:

(i) reflects the structural features of these enzymes better than their sole substrate specificity,
(ii) helps to reveal the evolutionary relationships between these enzymes,
(iii) provides a convenient tool to derive mechanistic information,
(iv) illustrates the difficulty of deriving relationships between family membership and substrate specificity

*Selection of GH10 enzyme examples according to the invention*

Three GH10 xylanases selected from microorganisms with maximal phylogenetic distance show efficacy of GH10 enzymes in modifying food product mixtures comprising rye. A xylanase each obtained from the:

- Kingdom fungi, division *ascomycota,* genus *Fusarium,* species *Fusarium verticilloides*
- Kingdom bacteria, phylum *proteobacteria,* genus *Aeromonas,* species *Aeromonas punctata*
- Kingdom bacteria, phylum *firmicutes,* genus *Clostridium,* species *Clostridium thermocellum* also known as *Ruminiclostridium thermocellum.*

Despite a low sequence similarity, the three-dimensional folding, and the mode of hydrolysis and the activity pattern is conserved as is shown by the results presented in the examples: low activity on model substrates and high modifying activity in food products mixtures comprising rye.

"Rye" in the context of the invention means all products generated through processing rye grain encompassing flakes, milled products such as flour and products generated from the grain and said products processed by physical, chemical and/or biological procedures. Further, "rye" in the context of the invention also encompass mixtures of rye with other cereal or processed grain. Such other cereal may be selected from the group comprising wheat, barley, triticale, emmer, oat, corn, millet, sorghum, buckwheat, quinoa, amaranth, and rice. The rye ratio in respective rye mixtures comprises preferably 10 % or more, 20 % or more or 30 % or more, more preferably 40 % or more, 50 % or more or 60 % or more, most preferably 70 % or more or 80 % or more, especially preferred 90 % or more or 95 % or more rye.

Said "food product comprising rye" may be a food product, which has been cooked, steamed, extruded or otherwise heated. Accordingly, the method of the invention comprises preferably a heating step, wherein e.g. baking, steaming, cooking or otherwise heating is applied to produce the final food product.

In one embodiment of the invention, said food product comprising rye may be a baked food product. Examples are bread, typically in the form of loaves or rolls, French baguette-type bread, flat bread, pita bread, tortillas, cakes, pancakes, biscuits, cookies, pie crusts, crisp bread, cracker, pizza, samosa and the like. In one embodiment, said food product comprising rye may be a steamed food product. Examples are steamed bread, buns or dumplings. In one embodiment, said food product comprising rye may be a cooked food product. Examples are cooked dumplings and pasta. In a further embodiment, said food product comprising rye may be an extruded or coextruded food product. Examples are bars, flips, crackers, cookies or cereal flakes.

In one embodiment of the method of the invention, said composition comprising at least one GH10 enzyme is added during the mixing and/or blending of a primary food mixture comprising the food product ingredients. The composition comprising at least one GH10 enzyme may also be added prior to mixing and/or blending of the food product ingredients. For some applications, the composition comprising at least one GH10 enzyme may be added after mixing and/or blending of the primary food mixture comprising the food product ingredients.

Accordingly, in a preferred embodiment of the invention, the method for the preparation of a food product comprising rye comprises the steps of:

- preparing a primary food mixture;
- adding to said primary food mixture a composition comprising at least one GH10 enzyme; and
- processing said primary food mixture to produce said food product comprising rye, wherein said processing is treating the primary food mixture with heat.

In one embodiment, the invention relates to a method for the preparation of products comprising rye, said method comprising the step of mixing and or blending the GH10 enzyme and other improving compositions to generate a dough, a batter, a powder, a dry mixture of ingredients or any other form of a primary food mixture, which is used to produce the food product of the invention.

The rye added in the method and products of the invention is typically added as a flour, but can also be in the form of flakes, bran or grains.

Arabinoxylan is contained in several sorts of cereals. The following table shows an overview of the arabinoxylan content of cereals:

| Cereal | Arabinoxylan g/kg kernel d.w. |
|---|---|
| Wheat* | 59 |
| Barley* | 65 |
| Rye* | 86 |

(continued)

| Cereal | Arabinoxylan g/kg kernel d.w. |
|---|---|
| Triticale* | 111 |
| Corn* | 37 |
| Sorghum ** | 24 |
| Oat** | 97 |
| Rice*** | 89 |
| d.w. dry weight<br>* (Oloffs et al., 1999)<br>** (Knudsen 2014)<br>*** (Frølich et al., 2013) | |

In a further embodiment, the method of the invention can also be performed using another cereal, which comprises arabinoxylan or mixed-linkage beta-glucan, instead of rye. Such other cereal may be selected from the group comprising wheat, barley, triticale, emmer, oat, corn, millet, sorghum, buckwheat, quinoa, amaranth, and rice.

Suitably, the primary food mixture and/or the food product of the invention further comprises, besides rye, primary and secondary raw materials. Primary raw materials are for example selected from yeast, salt, water and structural major components such as other cereals. Secondary raw materials are materials which improve the dough or final product in one way or another, such as for example the taste or softness of the food product. In a preferred embodiment, the primary food mixture and/or the food product of the invention further comprises one or more food-improving agent as secondary raw materials, which are selected from the group consisting of enzymes, hydrocolloids, emulsifiers, oxidants, fats and lipids, flavors, (poly)saccharides including (poly)saccharide alcohols, proteins, salts and acids, leavening agents, milk and cheese products, and other food additives or a mixture thereof.

Said food-improving agents may improve the processing of the primary food mixture or the properties of the food product, such as the volume, texture, microstructure, nutrition value, tolerance, digestibility, stability, taste, odor, shelf life time and the like.

Non-exclusive examples for enzymes, which may be added as food-improving agents are selected from the group comprising alpha-amylases, beta-amylases, maltogenic amylases, proteinases, other xylanases, arabinofuranosidases, maltotetraohydrolases, glucose oxidases, oxidoreductases, glucanases, cellulases, transglutaminases, isomerases, lipases, phospholipases, lipooxygenases, pectinases or a mixture thereof.

Non-exclusive examples for hydrocolloids, which may be added as food-improving agents are selected from the group comprising xanthan, carboxymethyl cellulose (CMC), methyl cellulose (MC) and hydroxypropylmethyl cellulose (HPMC), gum arabic, locust bean gum and tara gum, konjac mannan, gum tragacanth, pectin, gelatin and carrageen.

Non-exclusive examples for emulsifiers, which may be added as food-improving agents are selected from the group comprising lecithin E322, monoglycerides E471, DATEM E472e, ACETEM E472a, LACTEM E472b, SSL E481, CSL E482, polyglycerol esters E475, and propylene glycol ester E477.

Non-exclusive examples for oxidants and reductants, which may be added as food-improving agents are selected from the group comprising azodicarbonamide, ascorbic acid, potassium iodate, calcium iodate, potassium bromate, glutathione and cysteine.

Non-exclusive examples for fats and lipids, which may be added as food-improving agents are selected from the group comprising omega-3 fatty acid, animal fats e.g. butter or lard, vegetable oil, or mono- and diglycerides of fatty acids.

Non-exclusive examples for flavors, which may be added as food-improving agents are disclosed in the EU Lists of Flavourings database of the European Food Safety Authority (EFSA) with currently >2500 entries (see https://webgate.ec.europa.eu/foods_system/main/?event=substances.search&substances.so rt.by=substanceName&substances.sort.order=DESC&substances.pagination=1).

Non-exclusive examples for (poly)saccharides and (poly)saccharide alcohols, which may be added as food-improving agents are selected from the group comprising starch, cellulose, hemicellulose, polydextrose, cyclodextrines, maltodextrines, inulin, beta-glucan, pectin, psyllium husk mucilage, galactomannans or gums, glucomannan or konjac gum, gum acacia (arabic), karaya, tragacanth, gellan, xanthan, agar-agar, alginate, carrageenan, chitin and chitosan, sucrose, glucose, dextrose, lactose, maltose and erythritol.

Non-exclusive examples for proteins, which may be added as food-improving agents are selected from the group comprising gluten proteins originating from cereals and pseudocereals, soy meal, animal proteins, and insect proteins.

Non-exclusive examples for salts and acids, which may be added as food-improving agents are selected from the group comprising calcium carbonate E170, sorbic acid E200, potassium sorbate E202, calcium sorbate E203, acetic acid E260, sodium acetate E262, calcium acetate E263, lactic acid E270, propionic acid E280, calcium propionate E282, ascorbic acid E300, lecithine E322, citric acid E330, sodium citrate E331, potassium citrate E332, calcium citrate E333, calcium ortho-phosphate E341, diphosphate E450 and calcium sulfate E516.

Non-exclusive examples for leavening agents, which may be added as food-improving agents are selected from the group comprising bicarbonate, monocalcium phosphate, disodium pyrophosphate, sodium aluminium phosphate, baking yeast and sourdough.

Non-exclusive examples for milk and cheese products, which may be added as food-improving agents are selected from the group comprising milk powder, buttermilk powder, low fat milk powder, yoghurt powder, curd cheese powder and lactoprotein.

The GH10 enzyme may be added to the primary food mixture or the food product in a form selected from the group consisting of a cell extract, a cell-free extract, a partially purified protein and a purified protein.

Moreover, the GH10 enzyme may be added to the primary food mixture or the food product in the form of a solution or as a solid - depending on the use and/or the mode of application and/or the mode of administration. The solid form can be either as a dried enzyme powder or as a granulated enzyme.

In one embodiment, the invention provides an enzyme composition for addition to the primary food mixture or the food product, said composition comprising at least one GH10 enzyme and optionally at least one formulating agent, excipient, stabilizer and/or a preservative. The formulation can be a liquid formulation, such as a solution, or a dry formulation, such as a powder or granulate.

Liquid enzyme formulations are e.g. selected from the group comprising glycerol or water.

In one embodiment, the enzyme composition comprises a stabilizer. Stabilizers may, without being limited to these examples, be selected from:

- salts such as sodium chloride, magnesium chloride, sodium sulfate and potassium sulfate,
- small solutes like ectoine,
- amino acids or proteins, such as histidine, glycine, arginine or BSA,
- polyols, polymers and (poly)saccharides, e.g. starch, oligosaccharides, maltodextrin, trehalose, lactose, maltose, cellobiose, sucrose, mannitol, sorbitol, dextran or PEG;
- surfactants such as gelatin, poloxamers Brij, octyl-glucopyranoside, palmitic acid, dipalmitylphosphatidylcholine, hydroxypropyl-β-cyclodextrin, polysorbate 20 or polysorbate 80,
- antioxidantia, such as DTT, EDTA, THPP and mercaptoethanol,
- polycations, such as polyethyleneimine, and
- polyanions such as polyacrylic acid.

In a further embodiment, the enzyme composition comprises a preservative. The preservative is e.g. methyl paraben, propyl paraben, benzoate, sorbate or other food approved preservatives or a mixture thereof.

In yet a further embodiment, the enzyme composition comprises at least one other agent selected from extenders, fillers, binders, flavor maskers, bitter blockers and activity enhancers.

Suitably, all agents used in the enzyme composition to be added to the primary food mixture or the food product are used in food grade.

The GH10 enzyme used in the methods of the present invention may be obtained from microorganisms of any genus or species. For purposes of the present invention, the term "obtained from" as used herein in connection with a given source shall mean that the polypeptide encoded by a nucleotide sequence is produced by the source or by a strain in which the nucleotide sequence from the source has been inserted. The enzymes of the invention may be extracellularly produced or may be intracellularly produced. In a preferred embodiment, the polypeptide obtained from a given source is secreted extracellularly. Accordingly, the GH10 enzyme used in the methods of the present invention may be produced from a natural, a recombinant or synthetic gene/polynucleotide sequence.

The GH10 enzyme used in the methods of the present invention may be from a bacterial source. For example, the GH10 enzyme may be obtained from a genus of Gram positive bacteria such as a *Bacillus, Streptococcus, Streptomyces, Staphylococcus, Enterococcus, Lactobacillus, Lactococcus, Clostridium, Herbinix, Herbivorax, Geobacillus,* or *Oceanobacillus,* or from a genus of Gram negative bacteria such as an *Aeromonas, Cellovibrio, Shewanella, Duganella, Cystobacter, Escherichia, Pseudomonas, Pseudoalteromonas, Sorangium, Colwellia, Campylobacter, Helicobacter, Flavobacterium, Fusobacterium, Thermotoga, Ilyobacter, Neisseria,* or *Ureaplasma.*

In a preferred embodiment, the GH10 enzyme used in the methods of the invention is obtained from a group of bacteria comprising the species *Clostridium aldrichii, Clostridium alkalicellulosi, Clostridium caenicola, Clostridium cellobioparum, Clostridium cellulolyticum, Clostridium cellulosi, Clostridium clariflavum, Clostridium hungatei, Clostridium josui, Clostridium leptum , Clostridium methylpentosum, Clostridium papyrosolvens, Clostridium sporospaeroides,*

*Clostridium stercorarium, Clostridium straminosolvens, Clostridium sufflavum, Clostridium termitidis, Clostridium thermosuccinogenes, Clostridium viride or Clostridium thermocellum, Herbinix hemicellulosilytica, Herbinix luporum* and *Herbivorax saccincola.*

In another preferred embodiment, the GH10 enzyme used in the methods of the invention is obtained from a group of bacteria comprising *Aeromonas caviae, Tolumonas lignilytica, Pseudomonas psychrotolerans, Cellvibrio japonicus, Cellvibrio mixtus, Shewanella japonica, Pseudomonas psychrotolerans, Pseudomonas oryzihabitans, Pseudoalteromonas atlantica, Sorangium cellulosum* and *Cystobacter ferrugineus.*

In another preferred embodiment, the GH10 enzyme is obtained from a group of bacteria comprising *Streptomyces achromogenes, Streptomyces avermitilis, Streptomyces mirabilis, Streptomyces hyaluromycini, Streptomyces coelicolor, Streptomyces griseus* and *Streptomyces lividans.*

The GH10 enzyme used in the methods of the present invention may also be a fungal polypeptide, and more preferably a yeast polypeptide such as from a yeast genus comprised in the group of *Candida, Kluyveromyces, Pichia, Saccharomyces, Schizosaccharomyces,* and *Yarrowia;* or more preferably from a genus of filamentous fungi comprised in the group of *Acremonium, Agaricus, Alternaria, Aspergillus, Aureobasidium, Botryospaeria, Ceriporiopsis, Chaetomidium, Chrysosporium, Claviceps, Cochliobolus, Coprinopsis, Coptotermes, Corynascus, Cryphonectria, Cryptococcus, Diplodia, Exidia, Filibasidium, Fusarium, Gibberella, Holomastigotoides, Humicola, Hypocrea, Irpex, Lentinula, Leptospaeria, Magnaporthe, Melanocarpus, Meripilus, Mucor, Myceliophthora, Neocallimastix, Neurospora, Paecilomyces, Penicillium, Phanerochaete, Piromyces, Poitrasia, Pseudoplectania, Pseudotrichonympha, Rhizomucor, Schizophyllum, Scytalidium, Talaromyces, Thermoascus, Thielavia, Tolypocladium, Trichoderma, Trichophaea, Verticillium, Volvariella,* and *Xylaria.*

In a preferred embodiment, the GH10 enzyme used in the methods of the present invention is obtained from a group of yeasts comprising *Saccharomyces carlsbergensis, Saccharomyces cerevisiae, Saccharomyces diastaticus, Saccharomyces douglasii, Saccharomyces kluyveri, Saccharomyces norbensis* and *Saccharomyces oviformis.*

In another preferred embodiment, the GH10 enzyme used in the methods of the present invention is obtained from a group of fungi comprising *Acremonium cellulolyticus, Aspergillus aculeatus, Aspergillus awamori, Aspergillus fumigatus, Aspergillus foetidus, Aspergillus japonicus, Aspergillus nidulans, Aspergillus niger, Aspergillus oryzae, Chrysosporium keratinophilum, Chrysosporium lucknowense, Chrysosporium tropicum, Chrysosporium merdarium, Chrysosporium inops, Chrysosporium pannicola, Chrysosporium queenslandicum, Chrysosporium zonatum, Fusarium avenaceum, Fusarium bactridioides, Fusarium cerealis, Fusarium commune, Fusarium crookwellense, Fusarium culmorum, Fusarium fujikori, Fusarium graminearum, Fusarium graminum, Fusarium heterosporum, Fusarium mangiferae, Fusarium negundi, Fusarium oxysporum, Fusarium proliferatum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Fusarium verticilloides, Humicola grisea, Humicola insolens, Humicola lanuginosa, Irpex lacteus, Mucor miehei, Myceliophthora thermophila, Neurospora crassa, Penicillium funiculosum, Penicillium purpurogenum, Phanerochaete chrysosporium, Talaromyces cellulolyticus, Thermoascus aurantiacus, Thielavia achromatica, Thielavia albomyces, Thielavia albopilosa, Thielavia australeinsis, Thielavia fimeti, Thielavia microspora, Thielavia ovispora, Thielavia peruviana, Thielavia spededonium, Thielavia setosa, Thielavia subthermophila, Thielavia terrestris, Trametes versicolor, Trichoderma harzianum, Trichoderma koningii, Trichoderma longibrachiatum, Trichoderma reesei* and *Trichoderma viride.*

It will be understood that for the aforementioned genera and species the invention encompasses both the perfect and imperfect states, and other taxonomic equivalents, e.g., anamorphs, regardless of the species name by which they are known. Those skilled in the art will readily recognize the identity of appropriate equivalents.

Strains of these species are readily accessible to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

Furthermore, such polypeptides may be identified and obtained from other sources including microorganisms isolated from nature (e.g., soil, composts, water, etc.). Techniques for isolating microorganisms from natural habitats are well known in the art. Furthermore, the genes for such polypeptides may be isolated by activity screening of DNA-libraries prepared from cloning fragmented metagenomic DNA in suitable expression hosts such as *E. coli, Bacillus subtilis* or *Thermus thermophilus.* Screening of metagenomic libraries is a state of the art technology for obtaining new enzyme genes. The polynucleotide may then be obtained by similarly screening a genomic or cDNA library of such a microorganism or environmental sample. Once a polynucleotide encoding a polypeptide has been detected with suitable probe(s), the polynucleotide can be isolated or cloned by utilizing techniques that are well known to those of ordinary skill in the art (see, e.g., Sambrook et al., 1989).

In a preferred embodiment, the GH10 enzyme used in the methods of the present invention has xylanase and/or beta-glucanase activity. More preferably, the GH10 enzyme used in the methods of the present invention modifies the content of hemicellulose components. The GH10 enzyme alters the arabinoxylan content to overcome the limitations of products comprising rye e.g. compact, wet and sticky crumb structure as well as of bakery products with compact crumb and reduced loaf volume. In one embodiment, altering the arabinoxylan content means modifying the content of arabinoxylan. In another embodiment, altering the arabinoxylan content means changing the ratio of insoluble (WU-AX) to soluble (WE-AX) arabinoxylan.

In a further embodiment, the invention provides a primary food mixture comprising rye flour together with at least one GH10 enzyme.

In a further embodiment, the invention provides a food product comprising rye flour together with at least one GH10 enzyme.

The advantages and advantageous embodiments described above for the method equally apply to the primary food mixture and the food product of the invention, such that it shall be referred to the above.

The primary food mixture may contain other dough-improving and/or bread-improving additives, e.g. any of the additives mentioned above.

The present invention further provides polypeptides, which have the deduced amino acid sequence of SEQ ID NOs 1 to 6, as well as fragments, analogs and derivatives of such polypeptides. The terms "fragment", "derivative" and "analog", when referring to a polypeptide of SEQ ID NOs 1 to 6, means polypeptides that retain essentially the same biological function or activity as a xylanase. An analog might, for example, include a proprotein, which can be activated by cleavage of the proprotein to produce an active mature protein.

The polypeptides of the present invention may be recombinant polypeptides, natural polypeptides or synthetic polypeptides. The fragment, derivative or analog of a polypeptide of SEQ ID NOs 4 to 6, may be (i) one in which one or more of the amino acid residues is substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which additional amino acids are fused to the mature protein, such as a leader or secretory sequence or a sequence which is employed for purification, or for substrate or complex binding of the mature polypeptide, or a proprotein sequence. Such fragments, derivatives and analogs are deemed to be within the scope of those skilled in the art to provide upon the basis of the teachings herein.

The polypeptides of the present invention include the polypeptides of SEQ ID NOs 4 to 6, as well as polypeptides which have at least 75 % similarity (e.g. preferably at least 50 %; and more preferably at least 70 % identity) to a polypeptide of SEQ ID NOs 4 to 6, more preferably at least 85 % similarity (e.g. preferably at least 70 % identity) to a polypeptide of SEQ ID NOs 4 to 6, and most preferably at least 95 % similarity (e.g. preferably at least 90 % identity) to a polypeptide of SEQ ID NOs 4 to 6. Moreover, they should preferably include exact portions of such polypeptides containing a sequence of at least 30 amino acids, and more preferably at least 50 amino acids.

Fragments or portions of the polypeptides of the present invention may be employed as intermediates for producing the corresponding full-length polypeptides by peptide synthesis. Fragments or portions of the polynucleotides of the present invention may also be used to synthesize full-length polynucleotides of the present invention.

In a preferred embodiment, said GH10 enzyme comprises, essentially consists of or consists of a polypeptide which has at least 75 % amino acid sequence identity to a polypeptide selected from SEQ ID NOs 1 to 6 and which shows xylanase activity:

SEQ ID NO: 1: GH10 *(Clostridium thermocellum) WT*
SEQ ID NO: 2: GH10 *(Fusarium verticilloides) WT*
SEQ ID NO: 3: GH10 *(Aeromonas punctata) WT*
SEQ ID NO: 4: GH10 *(Clostridium thermocellum) w/o CBM*
SEQ ID NO: 5: GH10 *(Clostridium thermocellum) w/o doc*
SEQ ID NO: 6 GH10 *(Clostridium thermocellum) w/o doc/CBM*

In a more preferred embodiment, said GH10 enzyme with xylanase activity comprises, essentially consists of or consists of a polypeptide which has at least 75 % amino acid sequence identity to the polypeptide according to SEQ ID NO. 4, with the proviso that the GH10 enzyme is not the polypeptide of SEQ ID NO: 1, 2 or the polypeptide of SEQ ID NO: 3.

In a still more preferred embodiment, said GH10 enzyme comprises, essentially consists of or consists of a polypeptide having at least 75 % amino acid sequence identity to a polypeptide of SEQ ID NO: 4 to 6.

In a particularly preferred embodiment, said GH10 enzyme comprises, essentially consists of or consists of a polypeptide having at least 75 % amino acid sequence identity to a polypeptide of SEQ ID NO: 4, wherein in said GH10 enzyme the CBM is deleted which results in an 4 fold enzyme activity increase.

In a further particularly preferred embodiment, said GH10 enzyme comprises, essentially consists of or consists of a polypeptide having at least 75 % amino acid sequence identity to a polypeptide of SEQ ID NO: 5, wherein in said GH10 enzyme, the dockerin module is deleted, which results in an 4 fold enzyme activity increase.

In a further particularly preferred embodiment, said GH10 enzyme comprises, essentially consists of or consists of a polypeptide having at least 75 % amino acid sequence identity to a polypeptide of SEQ ID NO: 6, wherein in said GH10 enzyme, the CBM and the dockerin module are deleted, which results in an 8 fold enzyme activity increase.

Preferred according to the invention is a GH10 enzyme of any of SEQ ID NOs: 1-3, which displays optimal enzyme activity, in particular xylanase activity, at an acidic pH preferably in the range of 3.5 to 6.5, preferably in the range of 3.5 to 6.0, more preferably 3.5 to 5.5 or 3.5 to 5.0, most preferably in the range of 3.5 to 4.5, such as 4.3.

Such enzymes are especially suitable for use in the processing of the primary food mixtures of the invention, which are heat-treated to form the final food products. The GH10 enzymes provided by the invention still display a sufficient xylanase activity to modify the arabinoxylan content or chain length of e.g. a dough comprising rye also at higher temperatures. The pH optimum in the acidic range is advantageous for example also in the processing of sour doughs.

The method and the GH10 enzyme of the present invention are advantageous in that it leads, for example, to dough improvement, especially of rye comprising dough. Dough improvement means improving dough processing and/or improving the quality of final food products, such that the final food products show a less compact structure, an increased softness, a volume increase and/or a softer crumb structure.

The invention further relates to a nucleic acid molecule comprising a nucleic acid sequence encoding the GH10 enzyme according to the invention, in particular encoding an amino acid sequence selected from SEQ ID NOs 4 to 6.

The "polynucleotides" or "nucleic acids" of the present invention may be in the form of RNA or in the form of DNA; DNA should be understood to include cDNA, genomic DNA, recombinant DNA and synthetic DNA. The DNA may be double-stranded or single-stranded and, if single stranded, may be the coding strand or non-coding (antisense) strand. The coding sequence, which encodes the polypeptide may be identical to the coding sequence for the polypeptides shown in SEQ ID NOs: 1 to 6, preferably of SEQ ID NOs: 4 to 6, or it may be a different coding sequence encoding the same polypeptide, as a result of the redundancy or degeneracy of the genetic code or a single nucleotide polymorphism. For example, it may also be an RNA transcript which includes the entire length of coding sequence for a polypeptide of any one of SEQ ID NOs 4 to 6. In a preferred embodiment, the "polynucleotide" according to the invention is one of SEQ ID NOs 16 to 18.

The nucleic acids which encode the polypeptides of SEQ ID NOs: 1 to 6, preferably of SEQ ID NOs: 4 to 6 may include but are not limited to the coding sequence for the polypeptide alone; the coding sequence for the polypeptide plus additional coding sequence, such as a leader or secretory sequence or a proprotein sequence; and the coding sequence for the polypeptide (and optionally additional coding sequence) plus non-coding sequence, such as introns or a non-coding sequence 5' and/or 3' of the coding sequence for the polypeptide.

Thus, the term "polynucleotide encoding a polypeptide" or the term "nucleic acid encoding a polypeptide" should be understood to encompass a polynucleotide or nucleic acid which includes only a coding sequence for a GH10 enzyme of the invention, e.g. a polypeptide selected from SEQ ID NOs: 1 to 6, preferably of SEQ ID NOs: 4 to 6 as well as one which includes additional coding and/or non-coding sequence. The terms polynucleotides and nucleic acid are used interchangeably.

The present invention also includes polynucleotides where the coding sequence for the polypeptide may be fused in the same reading frame to a polynucleotide sequence which aids in expression and secretion of a polypeptide from a host cell; for example, a leader sequence which functions as a secretory sequence for controlling transport of a polypeptide from the cell may be so fused. The polypeptide having such a leader sequence is termed a preprotein or a preproprotein and may have the leader sequence cleaved, by the host cell to form the mature form of the protein. These polynucleotides may have a 5' extended region so that it encodes a proprotein, which is the mature protein plus additional amino acid residues at the N-terminus. The expression product having such a prosequence is termed a proprotein, which is an inactive form of the mature protein; however, once the prosequence is cleaved, an active mature protein remains. The additional sequence may also be attached to the protein and be part of the mature protein. Thus, for example, the polynucleotides of the present invention may encode polypeptides, or proteins having a prosequence, or proteins having both a prosequence and a presequence (such as a leader sequence).

The polynucleotides of the present invention may also have the coding sequence fused in frame to a marker sequence, which allows for purification of the polypeptides of the present invention. The marker sequence may be an affinity tag or an epitope tag such as a polyhistidine tag, a streptavidin tag, a Xpress tag, a FLAG tag, a cellulose or chitin binding tag, a glutathione-S transferase tag (GST), a hemagglutinin (HA) tag, a c-myc tag or a V5 tag.

The HA tag would correspond to an epitope obtained from the influenza hemagglutinin protein (Wilson et al., 1984), and the c-myc tag may be an epitope from human Myc protein (Evans et al., 1985).

The present invention is considered to further provide polynucleotides which hybridize to the hereinabove-described sequences wherein there is at least 70 %, preferably at least 90 %, and more preferably at least 95 % identity or similarity between the sequences, and thus encode proteins having similar biological activity. Moreover, as known in the art, there is "similarity" between two polypeptides when the amino acid sequences contain the same or conserved amino acid substitutes for each individual residue in the sequence. Identity and similarity may be measured using sequence analysis software (e.g., ClustalW at PBIL (Pole Bioinformatique Lyonnais) http://npsa-pbil.ibcp.fr). The present invention particularly provides such polynucleotides, which hybridize under stringent conditions to the hereinabove-described polynucleotides.

Suitably stringent conditions can be defined by, e.g., the concentration of salt or formamide in the prehybridization and hybridization solution, or by the hybridization temperature, and are well known in the art. In particular, stringency can be increased by reducing the concentration of salt, by increasing the concentration of formamide, and/or by raising the hybridization temperature.

For example, hybridization under high stringency conditions may employ about 50 % formamide at about 37 °C to 42 °C, whereas hybridization under reduced stringency conditions might employ about 35 % to 25 % formamide at about 30 °C to 35 °C. One particular set of conditions for hybridization under high stringency conditions employs 42 °C, 50 % formamide, 5x SSPE, 0.3 % SDS, and 200 μg/ml sheared and denatured salmon sperm DNA. For hybridization under reduced stringency, similar conditions as described above may be used in 35 % formamide at a reduced temperature of 35 °C. The temperature range corresponding to a particular level of stringency can be further narrowed by calculating the purine to pyrimidine ratio of the nucleic acid of interest and adjusting the temperature accordingly. Variations on the above ranges and conditions are well known in the art. Preferably, hybridization should occur only if there is at least 95 %, and more preferably at least 97 %, identity between the sequences. The polynucleotides which hybridize to the hereinabove described polynucleotides in a preferred embodiment encode polypeptides which exhibit substantially the same biological function or activity as the mature protein of SEQ ID NOs: 1 to 6, preferably of SEQ ID NOs: 4 to 6.

As mentioned, a suitable polynucleotide probe may have at least 14 bases, preferably 30 bases, and more preferably at least 50 bases, and will hybridize to a polynucleotide of the present invention, which has an identity thereto, as hereinabove described. For example, such polynucleotides may be employed as a probe for hybridizing to the polynucleotides encoding the polypeptides of SEQ ID NOs: 4 to 6, such as the polynucleotides of SEQ ID NOs 16-18, respectively, for example, for recovery of such a polynucleotide, or as a diagnostic probe, or as a PCR primer. Thus, the present invention includes polynucleotides having at least a 70 % identity, preferably at least a 90 % identity, and more preferably at least a 95 % identity to a polynucleotide of SEQ ID NOs 16 to 18, which encodes a polypeptide of SEQ ID NOs 4 to 6, as well as fragments thereof, which fragments preferably have at least 30 bases and more preferably at least 50 bases.

The terms "homology" or "identity," as used interchangeably herein, refer to sequence similarity between two polynucleotide sequences or between two polypeptide sequences, with identity being a more strict comparison. The phrases "percent identity or homology" and "identity or homology" refer to the percentage of sequence similarity found in a comparison of two or more polynucleotide sequences or two or more polypeptide sequences. "Sequence similarity" refers to the percent similarity in base pair sequence (as determined by any suitable method) between two or more polynucleotide sequences. Two or more sequences can be anywhere from 0-100 % similar, or any integer value there between. Identity or similarity can be determined by comparing a position in each sequence that can be aligned for purposes of comparison. When a position in the compared sequence is occupied by the same nucleotide base or amino acid, then the molecules are identical at that position. A degree of similarity or identity between polynucleotide sequences is a function of the number of identical or matching nucleotides at positions shared by the polynucleotide sequences.

A degree of identity of polypeptide sequences is a function of the number of identical amino acids at positions shared by the polypeptide sequences. A degree of homology or similarity of polypeptide sequences is a function of the number of amino acids at positions shared by the polypeptide sequences. The term "substantially identical," as used herein, refers to an identity or homology of at least 70 %, 75 %, at least 80 %, at least 85 %, at least 90 %, 91 %, 92 %, 93 %, 94 %, 95 %, 96 %, 97 %, 98 %, 99 %, or more.
The degree of sequence identity is determined by choosing one sequence as the query sequence and aligning it with the internet-based tool ClustalW with homologous sequences taken from GenBank using the blastp algorithm (NCBI).

As it is well known in the art, the genetic code is redundant in that certain amino acids are coded for by more than one nucleotide triplet (codon), and the invention includes those polynucleotide sequences which encode the same amino acids using a different codon from that specifically exemplified in the sequences herein. Such a polynucleotide sequence is referred to herein as an "equivalent" polynucleotide sequence. The present invention further includes variants of the hereinabove described polynucleotides which encode for fragments, such as part or all of the protein, analogs and derivatives of a polypeptide of SEQ ID NOs 4 to 6. The variant forms of the polynucleotide may be a naturally occurring allelic variant of the polynucleotide or a non-naturally occurring variant of the polynucleotide. For example, the variant in the nucleic acid may simply be a difference in codon sequence for the amino acid resulting from the degeneracy of the genetic code, or there may be deletion variants, substitution variants and addition or insertion variants. As known in the art, an allelic variant is an alternative form of a polynucleotide sequence, which may have a substitution, deletion or addition of one or more nucleotides that does not substantially alter the biological function of the encoded polypeptide.

The present invention also includes vectors, which include such polynucleotides, host cells which are genetically engineered with such vectors and the production of the polypeptides of SEQ ID NOs: 1 to 6, preferably of SEQ ID NOs: 4 to 6 by recombinant techniques using the foregoing. Host cells are genetically engineered (transduced or transformed or transconjugated or transfected) with such vectors, which may be, for example, a cloning vector or an expression vector. The vector may be, for example, in the form of a plasmid, a conjugative plasmid, a viral particle, a phage, etc. The vector or the gene may be integrated into the chromosome at a specific or a not specified site. Methods for genome integration of recombinant DNA, such as homologous recombination or transposase-mediated integration, are well known in the art. The engineered host cells can be cultured in conventional nutrient media modified as appropriate for activating promoters, selecting transformants or amplifying the genes of the present invention. The culture conditions, such as temperature, pH and the like, are those commonly used with the host cell selected for expression, as well known to the ordinarily skilled artisan.

The polynucleotides of the present invention may be employed for producing the polypeptides of SEQ ID NOs: 1 to 6, preferably of SEQ ID NOs: 4 to 6 by recombinant techniques. Thus, for example, the polynucleotides may be included in any one of a variety of expression vectors.

The appropriate DNA sequence may be inserted into the vector by any of a variety of procedures. In general, the DNA sequence is inserted into an appropriate restriction endonuclease site(s) by procedures well known in the art, which procedures are deemed to be within the scope of those skilled in this art.

The DNA sequence in the expression vector is operatively linked to an appropriate expression control sequence(s) (promoter) to direct mRNA synthesis. As representative examples of such promoters, there may be mentioned: LTR or SV40 promoter, the *E. coli lac, ara, rha* or *trp,* the phage lambda $P_L$ promoter and other promoters known to control expression of genes in prokaryotic or eukaryotic cells or their viruses

More preferably, the GH10 enzymes of the invention can be expressed using the following tools:

Specific examples of suitable promoters for directing the transcription of the nucleic acid constructs of the present invention, especially in a bacterial host cell, are the promoters obtained from the *E. coli lac* operon, *Streptomyces coelicolor* agarase gene (*dagA*)*, Bacillus subtilis* levansucrase gene (*sacB*)*, Bacillus licheniformis* alpha-amylase gene (*amyL*)*, Bacillus stearothermophilus* maltogenic amylase gene (*amyM*)*, Bacillus amyloliquefaciens* alpha-amylase gene (*amyQ*)*, Bacillus licheniformis* penicillinase gene (*penP*)*, Bacillus subtilis* xylose based expression via *xylA* and *xylB* genes, *B. subtilis* $sigma^B$ dependent expression of general stress proteins (*gsiB*)*,* and prokaryotic beta-lactamase gene (Villa-Kamaroff et al., 1978), as well as the tac promoter (DeBoer et al., 1983). Furthermore, the constitutive promotors p43 of *B. subtilis* and hpall of *Staphylococcus aureus.* Further promoters are described in "Useful proteins from recombinant bacteria" in Scientific American, 1980, 242: 74-94; and in Sambrook et al., 1989. Also possible are hybrids, and double or triple combinations of the above mentioned promotors, as well as mutated and truncated variants thereof.

Examples of suitable promoters for directing the transcription of the nucleic acid constructs of the present invention in a filamentous fungal host cell are promoters obtained from the genes for *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase (*glaA*), *Rhizomucor miehei* lipase, *Aspergillus oryzae* alkaline protease, *Aspergillus oryzae* triose phosphate isomerase, *Aspergillus nidulans* acetamidase, *Fusarium venenatum* amyloglucosidase (WO 00/56900), *Fusarium venenatum* Daria (WO 00/56900), *Fusarium venenatum* Quinn (WO 00/56900), *Fusarium oxysporum* trypsin-like protease (WO 96/00787), *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase IV, *Trichoderma reesei* endoglucanase V, *Trichoderma reesei* xylanase I, *Trichoderma reesei* xylanase II, *Trichoderma reesei* beta-xylosidase, as well as the NA2-tpi promoter (a modified promoter from the gene encoding neutral alpha-amylase in *Aspergillus niger* in which the untranslated leader has been replaced by an untranslated leader from the gene encoding triose phosphate isomerase in *Aspergillus nidulans*); and mutant, truncated, and hybrid promoters thereof.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992. In a *Pichia* host, useful promoters are obtained from the genes for *Pichia pastoris* alcohol oxidase (AOX1) and *Pichia pastoris* glyceraldehyde 3-phosphate dehodrogenase (GAP).

The control sequence may also be a suitable transcription terminator sequence, a sequence recognized by a host cell to terminate transcription. The terminator sequence is operably linked to the 3' terminus of the nucleotide sequence encoding the polypeptide. Any terminator that is functional in the host cell of choice may be used in the present invention. Preferred terminator structures are for example from the *Bacillus amyloliquefaciens amyE* gene, the *Bacillus licheniformis penP* gene, the *B. subtilis bglS* or *apreE* gene, and the *Bacillus thuringiensis cry* gene.

Preferred terminators for filamentous fungal host cells are obtained from the genes for *Aspergillus oryzae* TAKA amylase, *Aspergillus niger* glucoamylase, *Aspergillus nidulans* anthranilate synthase, *Aspergillus* nigeralpha-glucosidase, and *Fusarium oxysporum* trypsin-like protease.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos et al., 1992.

The control sequence may also be a suitable leader sequence, a nontranslated region of an mRNA that is important for translation by the host cell. The leader sequence is operably linked to the 5' terminus of the nucleotide sequence encoding the polypeptide. Any leader sequence that is functional in the host cell of choice may be used in the present invention. Preferred nontranslated regions are from the *Bacillus amyloliquefaciens amyE* gene, the *Bacillus licheniformis penP* gene, the *B. subtilis bglS* or *apreE* gene, and the *Bacillus thuringiensis cry* gene.

Preferred leader sequences for filamentous fungal host cells are obtained from the genes for *Aspergillus oryzae* TAKA amylase and *Aspergillus nidulans* triose phosphate isomerase.

Suitable leader sequences for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'terminus of the nucleotide sequence and, when transcribed, is recognized by the host cell as a signal to add polyadenosine residues to transcribed mRNA. Any polyadenylation sequence that is functional in the host cell of choice may be used in the present invention.

Preferred polyadenylation sequences for filamentous fungal host cells are obtained from the genes for *Aspergillus oryzae* TAKA amylase, *Aspergillus niger* glucoamylase, *Aspergillus nidulans* anthranilate synthase, *Fusarium oxysporum* trypsin-like protease, and *Aspergillus niger* alpha-glucosidase.

Useful polyadenylation sequences for yeast host cells are described by Guo and Sherman, 1995.

The control sequence may also be a signal peptide coding sequence that encodes a signal peptide linked to the amino terminus of a polypeptide and directs the encoded polypeptide into the cell's secretory pathway. The 5' end of the coding sequence of the nucleotide sequence may inherently contain a signal peptide coding sequence naturally linked in the translation reading frame with the segment of the coding sequence that encodes the secreted polypeptide. Alternatively, the 5' end of the coding sequence may contain a signal peptide coding sequence that is foreign to the coding sequence. The foreign signal peptide coding sequence may be required where the coding sequence does not naturally contain a signal peptide coding sequence. Alternatively, the foreign signal peptide coding sequence may simply replace the natural signal peptide coding sequence in order to enhance secretion of the polypeptide. However, any signal peptide coding sequence that directs the expressed polypeptide into the secretory pathway of a host cell of choice, i.e., secreted into a culture medium, may be used in the present invention.

Effective signal peptide coding sequences for bacterial host cells are the signal peptide coding sequences obtained from the genes for *Bacillus* NCIB 11837 maltogenic amylase, *Bacillus stearothermophilus* alpha-amylase, *Bacillus licheniformis* subtilisin, *Bacillus licheniformis* beta-lactamase, *Bacillus stearothermophilus* neutral proteases (*nprT, nprS, nprM*), and *Bacillus subtilis prsA.* Further signal peptides are described by Simonen and Palva, 1993, and Brockmeier et al., 2006.

Effective signal peptide coding sequences for filamentous fungal host cells are the signal peptide coding sequences obtained from the genes for *Aspergillus oryzae* TAKA amylase, *Aspergillus niger* neutral amylase, *Aspergillus niger* glucoamylase, *Rhizomucor miehei* aspartic proteinase, *Humicola insolens* cellulase, *Humicola insolens* endoglucanase V, and *Humicola lanuginosa* lipase.

Useful signal peptides for yeast host cells are obtained from the genes for *Saccharomyces cerevisiae* alpha-factor and *Saccharomyces cerevisiae* invertase. Other useful signal peptide coding sequences are described by Romanos et al., 1992, supra.

The control sequence may also be a propeptide coding sequence that encodes a propeptide positioned at the amino terminus of a polypeptide. The resultant polypeptide is known as a proenzyme or propolypeptide (or a zymogen in some cases). A propeptide is generally inactive and can be converted to a mature active polypeptide by catalytic or autocatalytic cleavage of the propeptide from the propolypeptide. The propeptide coding sequence may be obtained from the genes for *Bacillus subtilis* alkaline protease (*aprE*), *Bacillus subtilis* neutral protease (nprT), *Saccharomyces cerevisiae* alpha-factor, *Rhizomucor miehei* aspartic proteinase, and *Myceliophthora thermophila* laccase (WO 95/33836).

Where both signal peptide and propeptide sequences are present at the amino terminus of a polypeptide, the propeptide sequence is positioned next to the amino terminus of a polypeptide and the signal peptide sequence is positioned next to the amino terminus of the propeptide sequence.

It may also be desirable to add regulatory sequences that allow the regulation of the expression of the polypeptide relative to the growth of the host cell. Examples of regulatory systems are those that cause the expression of the gene to be turned on or off in response to a chemical or physical stimulus, including the presence of a regulatory compound.

Regulatory systems in prokaryotic systems include the *lac, tac,* and *trp* operator systems. In yeast, the ADH2 system, GAL1 system or AOX1 system may be used. In filamentous fungi, the TAKA alpha-amylase promoter, *Aspergillus niger* glucoamylase promoter, and *Aspergillus oryzae* glucoamylase promoter may be used as regulatory sequences. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the nucleotide sequence encoding the polypeptide would be operably linked with the regulatory sequence.

Expression Vectors

The present invention also relates to recombinant expression vectors comprising a polynucleotide of the present invention, a promoter, and transcriptional and translational stop signals. The various nucleic acids and control sequences described herein may be joined together to produce a recombinant expression vector that may include one or more (several) convenient restriction sites to allow for insertion or substitution of the nucleotide sequence encoding the polypeptide at such sites. Alternatively, a polynucleotide sequence of the present invention may be expressed by inserting the nucleotide sequence or a nucleic acid construct comprising the sequence into an appropriate vector for expression. In creating the expression vector, the coding sequence is located in the vector so that the coding sequence is operably linked with the appropriate control sequences for expression.

The recombinant expression vector may be any vector (e.g., a plasmid or virus) that can be conveniently subjected to recombinant DNA procedures and can bring about expression of the nucleotide sequence. The choice of the vector will typically depend on the compatibility of the vector with the host cell into which the vector is to be introduced. The vectors may be linear or closed circular plasmids.

The vector may be an autonomously replicating vector, i.e., a vector that exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vectors of the present invention preferably contain one or more (several) selectable markers that permit easy selection of transformed, transfected, transduced, or the like cells. A selectable marker is a gene the product of which provides for biocide or viral resistance, resistance to heavy metals, prototrophy to auxotrophs, and the like (Kroll et al., 2009). These auxotrophies include but are not limited to disruptions or deletions in amino acid biosynthesis for alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine, respectively. These auxotrophies may also include but are not limited to disruptions or deletions in purine, pyrimidine or enzyme cofactor biosynthesis genes. The auxotrophic phenotype is complemented episomally comprising an intact and expressed version of the mutated gene causing the auxotrophy together with an expression cassette containing the gene of interest.

Examples of bacterial selectable markers are the dal genes from *Bacillus subtilis* or *Bacillus licheniformis,* or markers that confer antibiotic resistance such as ampicillin, kanamycin, chloramphenicol, or tetracycline resistance. Suitable markers for yeast host cells are ADE2, HIS3, LEU2, LYS2, MET3, TRP1, and URA3. Selectable markers for use in a filamentous fungal host cell include, but are not limited to, *amdS* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), sC (sulfate adenyltransferase), and *trpC* (anthranilate synthase), as well as equivalents thereof. Preferred for use in an *Aspergillus* cell are the *amdS* and *pyrG* genes of *Aspergillus nidulans* or *Aspergillus oryzae* and the *bar* gene of *Streptomyces hygroscopicus.*

The vectors of the present invention preferably contain an element(s) that permits integration of the vector into the host cell's genome or autonomous replication of the vector in the cell independent of the genome.

For integration into the host cell genome, the vector may rely on the polynucleotide's sequence encoding the polypeptide or any other element of the vector for integration into the genome by homologous or nonhomologous recombination. Alternatively, the vector may contain additional nucleotide sequences for directing integration by homologous recombination into the genome of the host cell at a precise location(s) in the chromosome(s). To increase the likelihood of integration at a precise location, the integrational elements should preferably contain a sufficient number of nucleic acids, such as 100 to 10,000 base pairs, preferably 400 to 10,000 base pairs, and most preferably 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding nucleotide sequences. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" is defined herein as a nucleotide sequence that enables a plasmid or vector to replicate in vivo.

Examples of bacterial origins of replication are the origins of replication of plasmids pBR322, pUC19, pACYC177, and pACYC184 permitting replication in *E. coli,* and pUB110, pE194, pTA1060, and pAMβ1 permitting replication in *Bacillus.*

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991). Isolation of the AMA1 gene and construction of plasmids or vectors comprising the gene can be accomplished according to the methods disclosed in WO 00/24883.

More than one copy of a polynucleotide of the present invention may be inserted into a host cell to increase production of the gene product. An increase in the copy number of the polynucleotide can be achieved by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, e.g., Sambrook et al., 1989, supra).

In a preferred embodiment, the invention provides a host cell expressing the GH10 enzyme according to one of SEQ ID NOs: 1 to 6, preferably of SEQ ID NOs: 4 to 6. More preferably, said host cell comprises the nucleotide molecule of the invention, which encodes for a polypeptide of SEQ ID NOs: 1 to 6, preferably of SEQ ID NOs: 4 to 6. Most preferably, said host cell is *E. coli* or *Bacillus subtilis.*

The present invention provides in a further embodiment a method for producing a GH10 enzyme of SEQ ID NOs: 1 to 6, preferably of SEQ ID NOs: 4 to 6, the method comprising culturing a host cell as described herein under conditions permitting the production of the enzyme, and recovering the enzyme from the culture.

Methods of Production

More preferably, the present invention provides methods of producing a polypeptide of the present invention, i.e. a GH10 enzyme of SEO ID NOs: 1 to 6, preferably of SEO ID NOs: 4 to 6, comprising: (a) cultivating a cell, which produces the polypeptide, under conditions conducive for production of the polypeptide; and (b) recovering the polypeptide. In a preferred aspect, the cell is of the genus *Bacillus.* In a more preferred aspect, the cell is *Bacillus subtilis or Bacillus licheniformis.*

The present invention also relates to methods of producing a polypeptide of the present invention, comprising: (a) cultivating a recombinant host cell, as described herein, under conditions conducive for production of the polypeptide; and (b) recovering the polypeptide.

The present invention also relates to methods of producing a polypeptide of the present invention, comprising: (a) cultivating a recombinant host cell under conditions conducive for production of the polypeptide, wherein the host cell comprises a gene for a GH10 enzyme, more specifically a GH10 enzyme obtained from a bacterium, more specifically a recombinant bacterial GH10 enzyme.

In the production methods of the present invention, the cells are cultivated in a nutrient medium suitable for production of the polypeptide using methods well known in the art. For example, the cell may be cultivated by shake flask cultivation, and small-scale or large-scale fermentation (including continuous, batch, fed-batch, or solid-state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the polypeptide is secreted into the nutrient medium, the polypeptide can be recovered directly from the medium. If the polypeptide is not secreted into the medium, it can be recovered from cell lysates.

The polypeptides may be detected using methods known in the art that are specific for the polypeptides. These detection methods may include use of specific antibodies, formation of an enzyme product, or disappearance of an enzyme substrate. For example, an enzyme assay may be used to determine the activity of the polypeptide as described herein.

The resulting polypeptide may be recovered using methods known in the art. For example, the polypeptide may be recovered from the nutrient medium by conventional procedures including, but not limited to, centrifugation, filtration, extraction, spray-drying, evaporation, or precipitation.

The polypeptides of the present invention may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction (see, e.g., Protein Purification, J.-C. Janson and Lars Ryden, editors, VCH Publishers, New York, 1989) to obtain substantially pure polypeptides.

Compositions

The present invention also relates to compositions comprising a polypeptide of the present invention, i.e. a GH10 enzyme of SEQ ID NOs: 1 to 6, preferably of SEQ ID NOs: 4 to 6. Preferably, the compositions are enriched in such a polypeptide. The term "enriched" indicates that the GH10 xylanase activity of the composition has been increased, e.g., with an enrichment factor of at least 1.1.

The polypeptide compositions may be prepared in accordance with methods known in the art and may be in the form of a liquid or a dry composition. The polypeptide to be included in the composition may be stabilized in accordance with methods known in the art and as described hereinbefore.

Enzyme Preparation

The invention further provides an enzyme preparation comprising a GH10 enzyme of SEQ ID NOs: 1 to 6, preferably of SEQ ID NOs: 4 to 6, for use as a baking, cooking, steaming or extrusion additive in the process of the invention. The enzyme preparation is preferably in the form of a liquid, granulate or agglomerated powder, more preferably in the form of a granulate or agglomerated powder.

The granulate or agglomerated powder has preferably a narrow particle size distribution with more than 95 % (by weight) of the particles in the range from 25 to 500 micrometer.

Granulates and agglomerated powders may be prepared by conventional methods, e.g. by spraying the xylanase onto a carrier in a fluid-bed granulator. The carrier may consist of particulate cores having a suitable particle size. The carrier may be soluble or insoluble, e.g. a salt (such as sodium chloride or sodium sulfate), a sugar (such as sucrose or lactose), a sugar alcohol (such as sorbitol), an oligosaccharide (such as maltodextrin), starch, rice, corn grits, or soy.

In yet a further embodiment, the invention relates to the use of a GH10 enzyme in the production of food products comprising arabinoxylan.

In general, the advantages and advantageous embodiments described above for the method, primary food mixture and food product of the invention equally apply to the uses and methods of use according to the invention, such that it shall be referred to the above.

In particular, the present invention is also directed to methods of using a GH10 enzyme, such as GH10 enzyme of SEQ ID NOs: 1 to 6, preferably of SEQ ID NOs: 4 to 6, or compositions comprising the same in baking applications to generate a baked food product.

The present invention is also directed to methods of using a GH10 enzyme, such as GH10 enzyme of SEQ ID NOs: 1 to 6, preferably of SEQ ID NOs: 4 to 6, or compositions comprising the same in cooking applications to generate a steamed food product.

The present invention is also directed to methods of using a GH10 enzyme, such as GH10 enzyme of SEQ ID NOs: 1 to 6, preferably of SEQ ID NOs: 4 to 6, or compositions comprising the same in cooked applications to generate a cooked food product.

The present invention is also directed to methods of using a GH10 enzyme, such as GH10 enzyme of SEQ ID NOs: 1 to 6, preferably of SEQ ID NOs: 4 to 6, or compositions comprising the same in extrusion to generate an extruded food product.

The present invention is further described by the following figures and examples that should not be construed as limiting the scope of the invention.

Herein,

Figure 1 shows the relative enzyme activity increase (%) of SEQ ID NOs 4 to 6 enzymes in relation to the enzyme activity of the wildtype enzyme of SEQ ID NO 1. Surprisingly, the enzyme activity of the SEQ ID NOs 4 to 6 enzymes increases 4-8 fold in comparison to enzyme activity of the SEQ ID NO 1 enzyme. Enzyme activity: the release of micromol reducing sugars from arabinoxylan per mg of enzyme per minute was determined with the DNSA assay. Enzyme activity was measured in triplicates.

Figure 2 shows an SDS-PAGE of recombinantly produced enzymes of SEQ ID NOs 1, 4, 5 and 6, respectively. PageRuler Prestained Protein Ladder 10 to 180 kDa (#26616, ThermoFischer Scientific) was used as protein standard.

Figure 3 shows the relative enzyme activity of GH10 enzymes of SEQ ID NOs 2 and 3 compared to the GH family 11 enzymes SEQ ID NO 7 and Pentopan Mono BG*. The activity of the SEQ ID NO 6 xylanase was set to 100 %. Enzyme activity: the release of micromol reducing sugars from arabinoxylan per mg of enzyme per minute was determined with the DNSA assay. Enzyme activity was measured in triplicates.

Figure 4 shows the results of using GH10 enzymes SEQ ID NOs 2, 3 and 6, compared to the GH family 11 enzymes of SEQ ID NO 7 and Pentopan Mono BG*, on the bread volume in a method of baking rye bread. The enzyme concentration used was 0.1 ppm each. Baking and volume estimations were made in triplicates. The standard bread volume w/o enzyme addition was set to 100 %. Means with different letters are significantly different (One-Way ANOVA followed by Tukey test, $p<0.05$, $n=3$).

Figure 5 shows a photograph of rye bread, baked at a pH of 4.3 with 0.13 ppm GH10 enzyme of SEQ ID NO 6, compared to the standard w/o addition of a GH10 enzyme

Figure 6 shows the results of using 0.13 ppm GH10 enzyme of SEQ ID NO 6, compared to the standard w/o addition of a GH10 enzyme, on the crumb hardness in a method of baking rye bread at a pH of 4.3. Hardness calculations were made in duplicates.

Figure 7 shows the impact of different xylanase quantities 0.0 ppm (■), 0.01 ppm (□) 0.1 ppm (▦), on the stability of rye-wheat (ratio of rye (R) to wheat (W) 70:30 %) dough samples in a torque-measuring Z-kneader. Means with different letters are significantly different (One-Way ANOVA followed by Tukey test, $p<0.05$, $n=2$).

Figure 8 shows the impact of different xylanase quantities 0.0 ppm (■), 0.01 ppm (□) 0.1 ppm (▦), on the stickiness of rye-wheat (ratio of rye (R) to wheat (W) 30:70 %) dough samples determined by using the Ta.XT plus Texture Analyzer (Stable Micro Systems Ltd., Godalming, UK) equipped with a Chen-Hoseney Dough Stickiness Rig. Means with different letters are significantly different (One-Way ANOVA followed by Tukey test, $p<0.05$, $n=2$).

Figure 9 shows the impact of different xylanase quantities 0.0 ppm (■), 0.01 ppm (□) 0.1 ppm (▦), on the resistance to extension $R^K_{max}$ of rye-wheat (ratio of rye (R) to wheat (W) A) 10:90%, B) 30:70%, and C) 70:30 %) dough samples determined by using the Ta.XT plus Texture Analyzer (Stable Micro Systems Ltd., Godalming, UK) equipped with a SMS/Kieffer Extensibility Rig. Means with different letters are significantly different (One-Way ANOVA followed by Tukey test, $p<0.05$, $n=2$).

**Example 1: Cloning of SEQ ID NOs 14-19 encoding xylanases**

*Materials*

Chemicals used as buffers and substrates were commercial products of at least reagent grade. *Escherichia coli* DH10B was used for routine cloning and *E. coli* BL21 Star (DE3) for expression of *Clostridium thermocellum* DSM1237 (also known as *Ruminiclostridium thermocellum* DSM1237) and *Herbivorax saccincola* DSM101079 xylanase genes of SEQ ID NO 14 an SEQ ID NOs 16-19 respectively. Synthetic DNA for cloning was received from Integrated DNA Technologies (Leuven, Belgium). Expression of *Fusarium verticilloides* xylanase of SEQ ID NO 15, *Pichia pastoris X33* (ThermoFisher) was used. *Aeromonas punctata* GH10 xylanase of SEQ ID NO 3 was purchased from Megazyme (E-XYNAP, Megazyme, Bray, Ireland) and Pentopan Mono BG* was purchased from Sigma-Aldrich (Sigma Aldrich, St. Louis, Missouri, USA)

*DNA modification*

Preparation of chromosomal and plasmid DNA, endonuclease digestion, and ligation were carried out by standard procedures (Sambrook J and Russell DW. 2001).

*Cloning of genes encoding GH10 and GH11 xylanases*

The genes with sequences of SEQ ID NOs 14, 16 to 18 encoding GH10 xylanases were amplified from chromosomal DNA from *C. thermocellum* DSM1237 in accordance with manufacturer's instructions (Phusion High-Fidelity DNA Polymerase, F530S, ThermoFisher Scientific) using primer set of SEQ ID NOs: 8 and 9 and SEQ ID NOs: 10 and 9, primer set SEQ ID NOs 8 and 11 and primer set SEQ ID Nos 10 and 11, respectively. With the SEQ ID NO 4 to 6 enzymes, the effect of different protein module deletions on enzyme activity and function was investigated. The cellulose binding domain was deleted in the SEQ ID NO 4 enzyme. The dockerin module was deleted in the SEQ ID NO 5 enzyme and both modules were deleted in in the SEQ ID NO 6 enzyme. All PCR products were subsequent cloned by Gibson assembly (NEB, Cat. Nr. E2611S) in *Nde*I/*Xho*I digested pET24c(+) vector (Novagen, MerckMillipore) and sequenced by Eurofins to confirm the correct sequence.
The DNA coding for sequence SEQ ID NO 15 of *Fusarium verticilloides* GH10 xylanase was deduced from the amino acid sequence disclosed in WO2014019219A1. DNA was synthesized by Integrated DNA Technologies, inserted in *Sal*I/*Eco*RI digested pICZ$\alpha$ A (EasySelect™ Pichia Expression Kit) using Gibson assembly (NEB, Cat. Nr. E2611S) and sequenced by Eurofins to confirm the correct sequence.

The coding sequence SEQ ID NO 19 of GH11 xylanase from *Herbivorax saccincola* was amplified from the respective chromosomal DNA of strain DSM101079 employing primer pair of SEQ ID NOs: 12 and 13. A new cellulose degrading bacterial strain named *Herbivorax saccincola* was isolated from a 20 l fermenter operated with cow manure and fed with maize silage at 55 °C (Koeck et al. 2016). For genome sequencing of the strain *Herbivorax saccincola,* a total of 4 microgram genomic DNA was used to construct an 8-k mate-pair sequencing library (Nextera Mate Pair Sample Preparation Kit, Illumina Inc.), which was sequenced applying the paired-end protocol on an Illumina MiSeq system. Analysis and interpretation of the *Herbivorax saccincola* genome sequence within GenDB and by means of the Carbohydrate-active-enzyme database dbCAN (Yin et al., 2012) revealed more than 100 genes predicted to encode enzymes that mainly belong to different families of Glycoside Hydrolases (GH) and Carbohydrate-Binding Modules (CBM). One of the enzymes (SEQ ID NO 7) was identified as glycoside hydrolase family 11 member.

*Construction of Pichia pastoris X33 recombinant strains expressing the SEO ID NO 15 xylanase.*

To transform *P. pastoris* strain X33 pICZalpha A containing the nucleic acid of SEQ ID NO 15 was linearized with *Sac*I and used to transform electro competent *P. pastoris* cells (Lin-Cereghino *et al.,* 2005). Clones growing on antibiotics containing YPDS Agar (1 % (wt/vol) yeast extract, 2 % (wt/vol) peptone and 2 % (w/v) dextrose, 1 M sorbitol and 1.5% (wt/vol) agar) were screened for insertion of the nucleic acid of SEQ ID NO 15 using colony PCR with primers provided in the EasySelect™ Pichia Expression Kit. Positive clones were selected for production of SEQ ID NO 2 enzyme.

**Example 2: Protein Production of the enzymes of SEQ ID NOs 1-7**

*Growth of cells*

Fed-batch fermentations of recombinant *E. coli* strains harbouring the GH10 Xylanase genes from *C. thermocellum* ATCC27405/DSM1237 of SEQ ID NOs 14, 16-18 and *Herbivorax saccincola* DSM101079 xylanase gene SEQ ID NO 19 were carried out in a 10 L Uni-Vessel controlled and equipped with a Biostat B Twin DCU (Sartorius AG, Göttingen, Germany). Temperature, pH, foam, turbidity, weight and dissolved oxygen were monitored online during fermentation. The dissolved oxygen (DO%) was set to 25 % (vol/vol) and maintained with increasing agitation and constant air flow. The formation of foam was controlled by the addition of Antifoam 206 (Sigma Aldrich, St. Louis, Missouri, USA). A pH of 6.9 was maintained by addition of a 25 % (vol/vol) ammonium hydroxide solution and 25 % (vol/vol) $HPO_4$ solution. *E. coli* strains were cultivated in Riesenberg medium (Korz et al., 1995) at the 10 L scale, the feeding solution consists of 1021 g/L glycerol, 20 g/L $MgSO_4 \cdot 7\ H_2O$, 13 mg/L EDTA, 4 mg/L $CoCl_2 \cdot 6\ H_2O$, 23.5 mg/L $MnCl_2 \cdot 4\ H_2O$, 2.5 mg/L $CuC1_2 \cdot 2\ H_2O$, 5 mg/L $H_3BO_3$, 4 mg/L $Na_2MoO_4 \times 2\ H_2O$, 16 mg/L $Zn(CH_3COO)_2 \cdot 2\ H_2O$, 40 mg/L Fe(III)citrate (Korz et al., 1995). After the consumption of the initial carbohydrate substrate, growth rate was controlled according to EQUATION 1, whereby ms, is the mass flow of substrate (g $h^{-l}$), $\mu_{set}$ the desired specific growth rate ($h^{-l}$), $Y_{X/S}$ the biomass/substrate yield coefficient (g $g^{-1}$), m the specific maintenance coefficient (g $g^{-1}$ $h^{-l}$), V the cultivation volume (L), and X the biomass concentration (g $L^{-1}$):

EQUATION 1

$$m_S = \left( \frac{\mu_{set}}{Y_{X/S}} + m \right) \cdot V(t) \cdot X(t) \cdot e^{\mu_{set}(t - t_F)}$$

The inoculation procedure was the following: Based on a cryo-stock, a fresh agar plate containing adequate antibiotics was prepared. With one colony an Erlenmeyer flask containing 30 mL Lysogeny Broth (Sambrook et al. 1989) was inoculated and incubated for 12 to 15 h at 30 °C. 30 mL of this first preculture was used to inoculate 500 mL of the fermentation medium in a 5 L Erlenmeyer flask and incubated for further 14 h. The 10 L fermenter was filled with 6 L fermentation medium and inoculated with 500 mL of the second preculture. Kanamycin was added at 50 μg/mL. Protein production was induced by changing the glycerol feed to lactose feed. Cells were harvested after 48 h by centrifugation for 1 h at 9000 rpm and 22 °C. Portions of 300 g cells were solved in 3 L lysis buffer (50 mM MOPS pH 7.3, 0.1 M NaCl, 20 mM imidazol). Cell lysis was achieved by ultrasonic treatment in a ultrasonic flow through chamber. Cell debris was separated by centrifugation (9000 rpm, 22 °C). Supernatant was clarified from residual cells or debris by tangential filtration applying a 0.2 μm filter cassette and three volumes washing with lysis buffer. The enzyme solution was concentrated employing tangential filtration with a 30 kDa filter cassette followed by dialysis with three volumes lysis buffer. GH10 xylanases were purified by immobilized metal ion affinity chromatography (IMAC). Pure enzymes were eluted with elution buffer containing 50 mM MOPS, pH 7.3, 0.25 M imidazole, 0.1 M NaCl, and 20 mM $CaCl_2$.

Another possibility for producing variants of the SEQ ID NO 1 enzyme, such as the enzymes of SEQ ID NOs 4-6 of the invention is to transform a competent *B. subtilis* strain with an appropriate vector comprising the mutated DNA and cultivating the recombinant strain in accordance with Park et al., 1991.

Production of *Fusarium verticilloides* xylanase of SEQ ID NO 2 was performed in *P. pastoris* X33 by inoculating a preculture harboring SEQ ID NO 15 genomic insertion in 5 mL YPD broth (1 % (wt/vol) Yeast extract, 2 % (w/v) peptone and 2 % (wt/vol) dextrose) in a 50 mL Erlenmeyer flask from a single colony grown on YPD agar (1 % (wt/vol) yeast extract, 2 % (wt/vol) peptone and 2 % (wt/vol) dextrose, 1.5 % (wt/vol) agar) and incubating the preculture for 6 h at 30 °C in an orbital shaker at 180 rpm. One ml of preculture was used to inoculate 300 ml expression culture in BMD 1 % medium (0.2M potassium phosphate buffer pH6, 13.4 g/L Yeast Nitrogen Base, 0.4 mg/l Biotin, 1.1 % (wt/vol) glucose) in a baffled 5 l Erlenmeyer flask and incubated for 64 h at 22°C in an orbital shaker at 180 pm. Expression was induced by addition of 240 ml BMM2 medium (0.2M Potassium Phosphate buffer pH 6, 13.4 g/L Yeast Nitrogen Base, 0.4 mg/l biotin, 1% (vol/vol) methanol) and transferred to 28°C. The expression culture was fed every 24 h with 9 % (vol/vol) BMM 10 medium (0.2M Potassium Phosphate buffer pH 6, 13.4 g/L Yeast Nitrogen Base, 0.4 mg/l Biotin, 5% (vol/vol) methanol) and 1% (vol/vol) pure methanol. The culture supernatant was used to purify the enzyme by immobilized metal ion affinity chromatography (IMAC) as described above.

**Example 3: Electrophoretic methods**

Sodium dodecyl sulphate (SDS) polyacrylamide gel electrophoresis was performed in accordance with Laemmli (1970). Proteins were resuspended in denaturating buffer and heated for 15 min at 95 °C. The PageRuler Prestained Protein Ladder 10 to 180 kDa (#26616, ThermoFischer Scientific) was used as molecular weight standard. The proteins were stained with Coomassie brilliant blue R-250 (Weber and Osbourne, 1969).

**Example 4: Protein Quantification**

The protein amount was determined by using Pierce™ BCA Protein Assay Kit (#23225, ThermoFischer Scientific) according to the instructions of the manufacturer.

**Example 5: Activity test, Arabinoxylan degradation**

*Xylanase activity*

For the purpose of the present invention, any of the commercially available xylanase activity measurement kits is suitable to determine xylanase activity. One suitable way of measuring the xylanase activity is as follows:

Xylanase activity measurement was performed at the temperature and pH optimum of the respective enzymes of SEQ ID NOs 1, 4, 5, 6 and 7 at 60 °C, pH 5.8 and SEQ ID NOs 2 and

Pentopan Mono BG* at 50 °C, pH 5.8 for 1 h with a final concentration of 0.86 % (wt/vol) arabinoxylan (wheat arabinoxylan, medium viscosity, Megazyme, Ireland), reaction buffer (100 mM MOPS, pH 6.5, 50 mM NaCl, 10 mM $CaCl_2$) and the appropriate amount of enzyme.

Quantification of reducing sugars was performed as described by Wood and Bhat (1988) using 3,5-dinitrosalicylic acid (DNSA). The amount of liberated reducing sugar ends was determined based on a calibration curve with glucose. One unit (U) is defined as the amount of enzyme required to liberate one μmole reducing sugar equivalents in one minute. All assays were performed at least in triplicates.

**Example 6: Characterization of the enzymes of SEQ ID NOs 4-6 in comparison to the enzyme of SEQ ID NO 1**

The *Clostridium thermocellum* GH10 xylanases (SEQ ID NOs: 1, 4-6) were produced, purified and quantified as described in Examples 1-4. Xylanase activity was determined as described in Example 5 with the exception that every enzyme was measured using a temperature and pH range in order to determine pH optimum (pH range) and temperature optimum (temperature range). The specific activity of the enzymes of SEQ ID NOs 1 and 4 to 6 were calculated as U/mg. The results were calculated as ratio to the wildtype enzyme SEQ ID NO 1. Figure 2 shows the size of the enzymes of SEQ ID Nos: 4 to 6 compared to the wildtype protein of SEQ ID NO 1. All variants of SEQ ID NOs 4 to 6 are between 12 and 36 % smaller compared to the wildtype protein. However, surprisingly the specific enzyme activities of the variants of SEQ ID NO 4, SEQ ID NO 5 and SEQ ID NO 6 are at least 4 fold higher (Fig. 1), which does not correspond to the size changes only (Fig. 2). Surprisingly, the deletion of protein domains as shown in the variants of SEQ ID NOs: 4 to 6 leads to a significant increase in enzyme activity. The individual domain deletions in the enzymes of SEQ ID NO 4 and 5 results in an approximately each 4 fold activity increase. The deletions of both domains in the enzyme of SEQ ID NO 6 leads to an even further specific enzyme activity increase which is not reflected by the size reduction of the enzyme.

**Example 7: Comparison GH11 and GH10 enzyme activity**

The GH11 enzyme of SEQ ID NO 7 and the GH10 enzymes of SEQ ID NOs 2 and 6 were cloned and produced as described in Examples 1 and 2. The respective protein concentration including the purchased GH11 enzyme Pentopan Mono BG* was determined as described in Example 4. Specific enzyme activity for SEQ ID NO 3 was provided by the manufacturer (Megazyme, Ireland). The specific enzyme activity calculated according to example 5 revealed very high specific enzyme activities of both GH11 enzymes, the enzyme of SEQ ID NO 7 and Pentopan Mono BG* in comparison to a rather low specific enzyme activities of GH10 enzymes of SEQ ID NOs 2, 3 and 6. In Figure 3 the results are shown as relative enzyme activity. The activity of the SEQ ID NO 6 xylanase was set to 100 %.

**Example 8: Baking experiments using GH10 and GH11 enzymes**

Baking experiments were performed to determine the effect of GH11 and GH10 enzymes on bread volume comprising rye flour (100%). In each case, 0.1 ppm enzyme in respect to rye flour was added in baking experiments according to the following recipe:

*Baking Recipe 1*

| | |
|---|---|
| Rye flour 120 g | (Type 1150, Rosenmühle) |
| Instant Dry Yeast (species *Saccharomyces cerevisiae*) | 1 % (based on flour amount) |
| Salt | 2 % (based on flour amount) |
| Sugar | 2 % (based on flour amount) |
| Water | 57 % (based on flour amount) |

The enzymes of SEQ ID NOs 2, 3, 6 and 7 as well as Pentopan Mono BG* were used, each 0.1 ppm based on rye flour.

*Procedure*

1. Scaling of ingredients, addition of flour, yeast, salt, sugar and enzyme
2. Enzyme was dosed 0.1 ppm in relation to flour; excl. the negative control (no enzyme)
3. Temperature adjustment of water in order to reach a dough temperature of 30 °C after mixing, scaling and addition of water into mixer bowl
4. Mixing: 1 min with an hand mixer (Philips) at full speed
5. The dough is given 30 min at 30 °C in an oven.
6. Mixing: 1 min with a hand mixer (Philips) at full speed
7. The dough is given 45 min at 30 °C in an oven.
8. Mixing: 1 min with an hand mixer (Philips) at full speed
9. Dough was divided in equally heavy portions and molded to rolls
10. The molded roll is given 10 min bench-time

11. The rolls are transferred to a covered baking sheet.
12. The rolls are baked for 25 min (220 °C heated from the top and the bottom)
13. The rolls are allowed to cool down
14. The rolls are evaluated

*Volume estimation*

The volume of baked rolls and bread was measured by rapeseed displacement. A suitable beaker (three times height, two times wide and two times length compared to the biggest test sample) was filled with rapeseeds until the beaker was completely filled. Surplus rapeseeds were removed with a plate, which completely covered the beaker hole. The test roll was placed on top on the rapeseeds in the middle of the beaker and was covered by the plate. By applying steady moderate pressure on the plate, the roll was plunged into the rapeseeds. Displaced rapeseeds were collected and measured in a measuring glass. Displaced ml of rapeseed were considered as rolls volume. For every baking experiment volume calculations were run in triplicates. Several xylanases belonging to families 10 and 11 obtained from a variety of species were tested. Figure 4 summarizes the baking experiments of five xylanases, two GH11 family representatives and three GH10 xylanases obtained from different bacteria or fungi. The same amount of the individual enzymes was used for the baking trials. The GH10 enzymes are characterized by a low specific activity on arabinoxylan, however these enzymes demonstrate the best increase in bread volume. As shown in Example 7, representatives of GH11 enzymes show a much higher specific enzyme activity compared to GH10 enzyme. However, it was surprisingly discovered that, using the same amounts of enzymes in baking experiments, GH11 enzymes failed to increase the bread volume.

**Example 9 Effects of the enzyme of SEQ ID NO 6 on pH adjusted rye bread**

Following a different baking recipe (2), including pH adjustment, the effects of the enzyme of SEQ ID NO 6 on rye bread volume and hardness were tested.

*Baking Recipe 2*

| | | |
|---|---|---|
| Rye flour | 100 % Type 997 | |
| Instant Dry Yeast | 1.8 % (species: *S. cerevisiae*) | (based on flour amount) |
| Salt | 1.8 % | (based on flour amount) |
| Water | 70 % | (based on flour amount) |
| Enzyme: | SEQ ID NO 6, 0.13 mg/kg flour (Control experiments w/o enzyme) (based on flour amount) | |
| Lactate | 0.0-2.0 ml for pH adjustment (4.3, 4.7, 5.1 and 5.9) (based on flour amount) | |

*Procedure*

1. Scaling of ingredients, addition of flour, yeast, salt and enzyme
2. Temperature adjustment of water in order to reach a dough temperature of 30 °C after mixing, scaling and addition of water into mixer bowl
4. Kneading: 3 min with a Z-kneader at 63 rpm
5. Dough resting occurred for 10 min at 30 °C in a proofing chamber
6. Each 200 g breads are molded and transferred into baking tins
7. Proofing occurred 75 min at 30 °C in a proofing chamber (90 % relative humidity).
8. The breads are baked for 5 min (230 °C from the top 200 °C from the bottom) and 0.5 l steam
9. The breads are baked for 7 min (200 °C from the top, 200 °C from the bottom)
10. The breads are baked for 7 min (200 °C from the top, 200 °C from the bottom)
11. The breads are chilled and stored at room temperature
12. The breads are evaluated

*Measurement of Crumb Structure*

Crumb Hardness was analyzed by means of a texture analyzer (TVT 300 XP, TexVol Instruments AB, Viken, Sweden) according to the AACC International method 2011.

The baking experiments were done in duplicates and demonstrated a volume increase in pH adjusted rye bread as shown in Figure 5. Compared to a baking control experiment w/o enzyme, the crumb structure was significantly improved. Figure 6 shows that using the enzyme of SEO ID NO 6 reduced the bread crumb hardness significantly.

**Example 10: Effect of the enzyme of SEQ ID NO 6 on rye-wheat dough in different flour ratios**

The effect of the enzyme of SEQ ID NO 6 on different dough qualities was investigated. Dough was prepared according to dough recipe 1 and analyzed as described in the following:

*Dough Recipe 3*

| | | |
|---|---|---|
| Rye flour | 10, 30 or 70 % | Type 997 |
| Wheat flour | 90, 70 or 30 % | Type 550 |
| Instant Dry Yeast | 1.8 % (Species: *S. cerevisiae*) | (based on flour amount) |
| Salt | 1.8% | (based on flour amount) |
| Water | 57 % | (based on flour amount) |
| Enzyme: | SEQ ID NO 6, was added in concentrations w/o enzyme, 0.01 and 0.1 ppm (based on flour amount) | |

In accordance with the American Association of Cereal Chemists (AACC) method 54-21.02 a torque measuring Z-kneader (doughLAB; Perten Instruments, Germany) was applied to prepare rye-wheat dough in ratios of 70:30 %, 30:70 %, and 10:90 % by using commercial rye flour type 997 and wheat flour type 550 (Rosenmühle, Landshut, Germany). To 100 parts flour mixture (moisture content corrected to 14.00 g/100 g flour) 57.0 parts de-mineralized water and different concentrations (0.01 or 0.1 ppm) of the xylanase SEQ ID NO 6 were added. Subsequently, kneading was performed for 153 s at 63 rpm and 30 °C.

*Methods*

1. Kneading properties determined by a Z-Kneader

In accordance to the mentioned AACC method 54-21.02 a torque measuring Z-kneader (doughLAB; Perten Instruments, Germany) was used to determine the dough stability, which describes the period of time between exceeding the 500 FU-line and the first drop under 500 FU and provides information about dough processability. All measurements were done by double identification.

As shown in Fig. 7, rising SEQ ID NO 6 xylanase quantities cause an increase of dough stability during kneading and enable an improved processability on dough with a rye ratio of 70 %.

2. Dough stickiness by Chen-Hoseney Dough Stickiness Cell

Analysis of dough stickiness was carried out by using the Ta.XT plus Texture Analyzer (Stable Micro Systems Ltd., Godalming, UK) equipped with a Dough Stickiness Rig as described by Chen and Hoseney (1995). The stickiness (g) of the dough was measured of a piece of dough extruded through a grid to a cylinder in polished Plexiglas (diameter 25 mm). Dough was placed into the cell after kneading and rested for 5 min at room temperature. The dough sample is compressed by the cylinder at a speed of 0.5 mm/s, an applied force of 40 g, and a contact time of 0.10 s; then the cylinder goes back to 4 mm at speed of 10 mm/s. All the measurements were done by double identification.

As shown in Fig. 8, by increasing the SEQ ID NO 6 xylanase quantity, the dough stickiness gets significantly reduced, which indicates an improved dough handling and machinability on dough with a rye ratio of 30 %.

3. Elongation properties by SMS/Kieffer Extensibility Rig

Uniaxial elongation was analyzed using the SMS/Kieffer Extensibility Rig for the Ta.XT plus Texture Analyzer (Stable Micro Systems Ltd., Godalming, UK). For this purpose, each dough sample was placed into the Kieffer sample plate. After 40 minutes equilibration time at 30 °C the maximum peak force (resistance to extension ($R_{Kmax}$)) of ten strands of each dough sample were recorded. The test settings were: pre-test speed: 2.00 mm/s; test speed: 3.3 mm/s; post-test speed: 10.0 mm/s; distance: 75 mm; trigger force: 5.0 g. All the measurements were done by double identification. As shown in Fig. 9, by increasing the SEQ ID NO 6 xylanase quantity, the dough resistance to extension gets significantly reduced, which improves loosening of dough during fermentation and enables higher bread volumes on dough with all tested rye ratios 10, 30 and 70 %

**References**

AACC International, 2011; AACC International. Approved methods of analysis, 11th ed. Method 74-10.02. Measurement of Bread Firmness-Compression Test. January 6, 2011. AACC International: St. Paul, MN, U.S.A.

AACC International, 2011; AACC International. Approved methods of analysis, 11th ed. Method 54-21.02. Measurement of Bread Firmness-Compression Test. January 6, 2011. AACC International: St. Paul, MN, U.S.A.

Adelsberger, H., Hertel, C., Glawischnig, E., Zverlov, V.V., Schwarz, W.H., 2004. Enzyme system of Clostridium stercorarium for hydrolysis of arabinoxylan: reconstitution of the in vivo system from recombinant enzymes. Microbiology 150 (7), 2257-2266.

Biely, P., Singh, S., Puchart, V., 2016. Towards enzymatic breakdown of complex plant xylan structures: state of the art. Biotechnol. Adv. 34 (7), 1260-1274.

Biely, P., Vrsanská, M., Tenkanen, M., Kluepfel, D., 1997. Endo-beta-1,4-xylanase families: differences in catalytic properties. Journal of biotechnology 57 (1-3), 151-166.

Bouraoui, H., Desrousseaux, M.-L., Ioannou, E., Alvira, P., Manai, M., Remond, C., Dumon, C., Fernandez-Fuentes, N., O'Donohue, M.J., 2016. The GH51 alpha-L-arabinofuranosidase from Paenibacillus sp. THS1 is multifunctional, hydrolyzing main-chain and side-chain glycosidic bonds in heteroxylans. Biotechnology for biofuels 9, 140.

Brockmeier, U., Caspers, M., Freudl, R., Jockwer A., Noll, T. and Eggert T., 2006, Systematic Screening of All Signal Peptides from Bacillus subtilis: A Powerful Strategy in Optimizing Heterologous Protein Secretion in Gram-positive Bacteria, Journal of Molecular Biology. Volume 362, Issue 3, Pages 393-402.

Chen, W. Z. and Hoseney, R.C., 1995. Development of an Objective Method for Dough Stickiness, In LWT - Food Science and Technology, Volume 28, Issue 5, Pages 467-473.

Collins, T., Gerday, C., Feller, G., 2005. Xylanases, xylanase families and extremphilic xylanases. FEMS Microbiology Reviews, 29: 3-23.

Courtin, C. M. and Delcour, J. A., 2002. Arabinoxylans and Endoxylanases in Wheat Flour Bread-making. Journal of Cereal Science 35 (2002) 225-243.

Courtin, C. M. Roelants, A. Delcour, J. A., 1999. Reconstitution Experiments Provide Insight into the Role of Endoxylanases in Bread-Making, Journal of Agricultural and Food Chemistry 47 (5), 1870-1877.

De Boer, H. A., Comstock, L. J., Vasser, M., 1983. The tac promoter: a functional hybrid derived from the trp and lac promoters. Proc. Natl Aca. Sci. USA 80: 21-25.

Döring, C., Hussein, M. A., Jekle, M., Becker, T., 2017. On the assessments of arabinoxylan localization and enzymatic modifications for enhanced protein networking and its structural impact on rye dough and bread, In Food Chemistry, Volume 229, Pages 178-187.

Döring C., Grossmann, I., Roth, M., Jekle, M., Koehler, P., Becker, T., 2017. Effect of rye bran particles on structure formation properties of rye dough and bread. Food Processing and Preservation. Journal of Food Processing and Preservation. Volume 41, Issue 4.

Evan, G. I., Lewis, G. K., Ramsay, G. and Bishop, J.M., 1985. Isolation of monoclonal antibodies specific for human c-myc proto-oncogene product. Mol Cell Biol. Dec; 5 (12):3610-6.

Gems, D., Johnstone, I. L. and Clutterbuck, A. J., 1991. An autonomously replicating plasmid transforms Aspergillus nidulans at high frequency. Gene. Feb 1; 98 (1):61-7.

Guo and Sherman, 1995. Molecular Cellular Biology 15: 5983-5990.

Izydorczyk, M. S., Biliaderis, C. G., 1995. Cereal arabinoxylans: advances in structure and physicochemical properties. Carbohydr. Polym., 28, 33-48.

Johnson, E.A., Sakajoh, M., Halliwell, G., Madia, A., Demain, A. L., 1982. Saccharification of Complex Cellulosic Substrates by the Cellulase System from Clostridium thermocellum Appl. Environ. Microbiol. 43(5), 1125-1132.

Knudsen, K. E. B., 2014. Fiber and nonstarch polysaccharide content and variation in common crops used in broiler diets. Poultry Science 93: 2380-2393.

Koeck, D.E., Mechelke, M., Zverlov, V.V., Liebl, W., Schwarz, W.H., 2016. Herbivorax saccincola gen. nov., sp. nov., a cellulolytic, anaerobic, thermophilic bacterium isolated via in sacco enrichments from a lab scale biogas reactor. International Journal of Systematic and Evolutionary Microbiolog. 66: 4458-4463.

Kolenová, K., Vršanská, M., Biely, P., 2006. Mode of endo-β, 4-xylanases of families 10 and 11 on acidic xylooligosaccharides. Journal of Biotechnology, 121:338-345.

Korz, D. J., Rinas, U., Hellmuth K., Sanders, E. A., Deckwer, W.D., 1995. Simple fed-batch technique for high cell density cultivation of Escherichia coli. J. Biotechnol. 39:59-65.

Kroll, J., Steinle, A., Reichelt, R., Ewering, C. and Steinbüchel, A., 2009. Establishment of a novel anabolism-based addiction system with an artificially introduced mevalonate pathway: Complete stabilization of plasmids as universal application in white biotechnology. Metabolic Engineering 11: 168-177.

Larson, S. B., Day, J., Barba de la Rosa, A. P., Keen, N. T., McPherson, A., 2003. First crystallographic structure of a xylanase from glycoside hydrolase family 5: implications for catalysis. Biochemistry, 42: 8411-8422.

Lin-Cereghino, J., Won, W. W., Xiong, S., Gian, W., Luong, L.T., Vu, J., Johnson, S.D. and Lin-Cereghino G.P., 2005. Condensed protocol for competent cell preparation and transformation of the methylotrophic yeast Pichia pastoris. Biotechniques. 38(1):44, 46, 48.

Lombard V., Golaconda R.H., Drula E., Coutinho P.M., Henrissat B., 2014. The Carbohydrate-active enzymes database (CAZy) in 2013. Nucleic Acids Res 42:D490-D495. [PMID: 24270786].

Meuser, F., Suckow, P., 1986. Non-starch polysaccharides. In Chemistry and Physics of Baking; Blanchard, J. M. V., Frazier, P. J., Galliard, T., Eds.; The Royal Society of Chemistry: London, U.K; pp 42-61.

Oloffs K, Jeroch H, Schöner FJ., 1999. Efficacy of non-starch polysaccharide hydrolyzing enzymes on nutrient digestibility and gross energy convertibility of barley-rye and wheat-rye based diets for laying hens. Arch_Tierernahr.; 52(2): 155-65.

Park, Y.S., Kai, K., Iijima, S., Kobayashi, T., 1991. Enhanced β-Galactosidase Production by High Cell-Density Culture of Recombinant Bacillus subtilis with Glucose Concentration control). Biotechnol Bioeng. 40(6), 686-696.

Romanos, M. A., Scorer, C. A. and Jeffrey, J. C., 1992. Foreign Gene Expression in Yeast: a Review. YEAST VOL. 8: 423-488.

Sambrook, J, Russell, D. W., 2001.Molecular cloning: a laboratory manual 3rd edition. Coldspring-Harbour Laboratory Press, UK.

Sambrook, J., Fritsch, E. F., Maniatis. T., 1989. Molecular cloning: a laboratory manual, 2nd ed. Cold Spring Harbor Laboratory Press, Cold Spring Harbor, NY.

Simonen, M. and Palva, I., 1993. Protein secretion in Bacillus species. Microbiological Reviews 57: 109-137.

Villa-Komaroff, L., Efstratiadis, A.,Broome, S., Lomedico, P., Tizard, R., Naber, S. P., Chick, W. L., Gilbert, W., 1978. A bacterial clone synthesizing proinsulin. Proc. Natl. Acad. Sci. USA 75: 3727-3731.

Wenche Frølich, W., Aman, P. and Inge Tetens, T., 2013. Whole grain foods and health a Scandinavian perspective.

Food & Nutrition Research, 57.

Wilson, I. A., Niman, H. L., Houghten, R. A., Cherenson, A. R., Conolly, M. L., Lerner, R. A., 1984. The structure of an antigenic determinant in a protein. Cell. ; 37(3):767-78.

Wood, T. M., Bhat, K. M., 1988. Methods for measuring cellulase activities. Methods in Enzymology. 160: Academic Press, 87-112.

Yin, Y.B., Mao, X.Z., Yang, J.C., Chen, X., Mao, F.L., Xu, Y., 2012. dbCAN: a web resource for automated carbohydrate-active enzyme annotation. Nucleic Acids Res 40, W445-W451.

## Claims

1. A method for the preparation of a food product comprising rye, said rye comprising arabinoxylan, and said method comprising the steps of:

   • preparing a primary food mixture;
   • adding to said primary food mixture a composition comprising at least one glycoside hydrolase family 10 (GH10) enzyme; and
   • processing said primary food mixture to produce said food product comprising rye;
   wherein the addition of said at least one GH10 enzyme results in dough improvement.

2. The method of claim 1, wherein said at least one GH10 enzyme has hemicellulolytic activity, preferably xylanolytic activity.

3. The method according to claim 1 or 2, wherein said processing means treating of said primary food mixture with heat, such as baking, steaming, cooking or otherwise heating.

4. The method according to any one of the preceding claims, wherein said composition comprising at least one GH10 enzyme is added during the mixing and/or blending of the primary food mixture.

5. The method according to any one of the preceding claims, wherein said composition comprising at least one GH10 enzyme further comprises another agent, which is selected from the group consisting of other enzymes, hydrocolloids, emulsifiers, oxidants, fats and lipids, flavors, (poly)saccharides, proteins, salts and acids, leavening agents, milk and cheese products or a mixture thereof.

6. The method according to any one of the preceding claims, wherein said at least one GH10 enzyme is added to said primary food mixture in a form selected from the group consisting of a cell extract, a cell-free extract, a partially purified protein and a purified protein.

7. The method according to any one of the preceding claims, wherein said composition comprising at least one GH10 enzyme further comprises an enzyme carrier and optionally a stabilizer and/or a preservative and/or another agent selected from extenders, fillers, binders, flavour maskers, bitter blockers and activity enhancers.

8. The method according to any one of the preceding claims, wherein said at least one GH10 enzyme is isolated from a microorganism and/or is a recombinant enzyme.

9. The method according to any one of the preceding claims, wherein said at least one GH10 enzyme modifies the content of hemicellulose components, in particular the arabinoxylan content in said food product and/or wherein dough improvement means improving dough processing and/or improving the quality of final food products, such that the final food products show a less compact structure, an increased softness, a volume increase, a homogeneous pore distribution and/or a softer crumb structure.

10. Use of a GH10 enzyme in the production of food products comprising arabinoxylan.

11. The method or use according to any one of the preceding claims, wherein said GH10 enzyme comprises, essentially consists of or consists of a polypeptide which has at least 75 % amino acid sequence identity to a polypeptide selected from SEQ ID NO: 1-6 and which shows hemicellulolytic activity.

**12.** A GH10 enzyme with hemicellulolytic activity, which comprises, essentially consists of or consists of a polypeptide which has at least 75 % amino acid sequence identity to the polypeptide according to SEQ ID NOs 4 to 6, with the proviso that the GH10 enzyme is not the polypeptide of SEQ ID NO: 1, 2 or the polypeptide of SEQ ID NO: 3.

**13.** A nucleic acid molecule comprising, consisting essentially of or consisting of a nucleic acid sequence of SEQ ID NO: 16, 17 or 18 encoding the GH10 enzyme according to claim 12.

**14.** An expression vector comprising the nucleic acid molecule of claim 13.

**15.** A host cell expressing the GH10 enzyme according to claim 12.

**Fig.1**

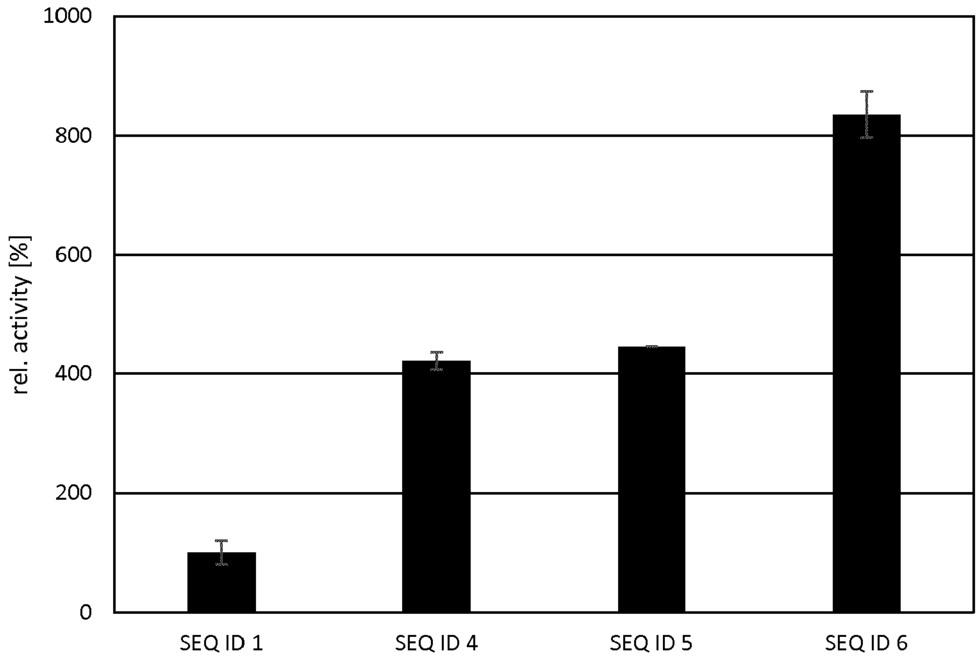
1000
800
600
400
200
0
rel. activity [%]
SEQ ID 1
SEQ ID 4
SEQ ID 5
SEQ ID 6

**Fig. 2**

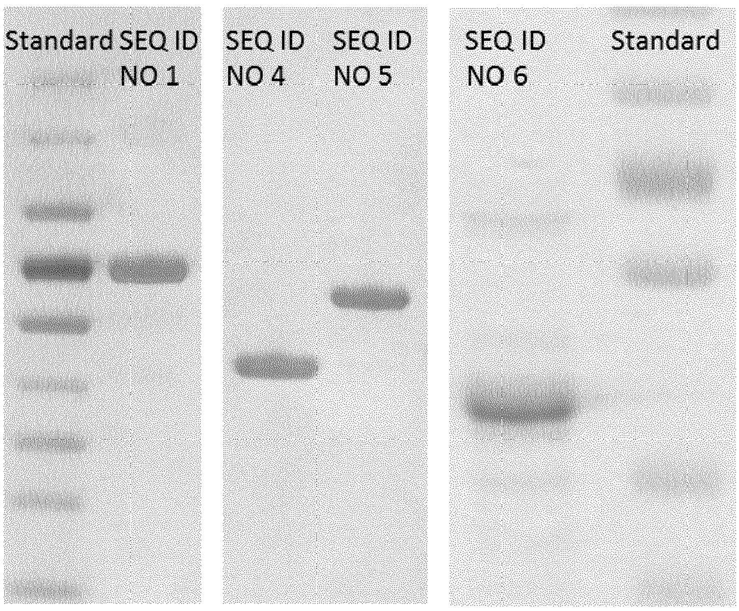
Standard
SEQ ID NO 1
SEQ ID NO 4
SEQ ID NO 5
SEQ ID NO 6
Standard

**Fig. 3**

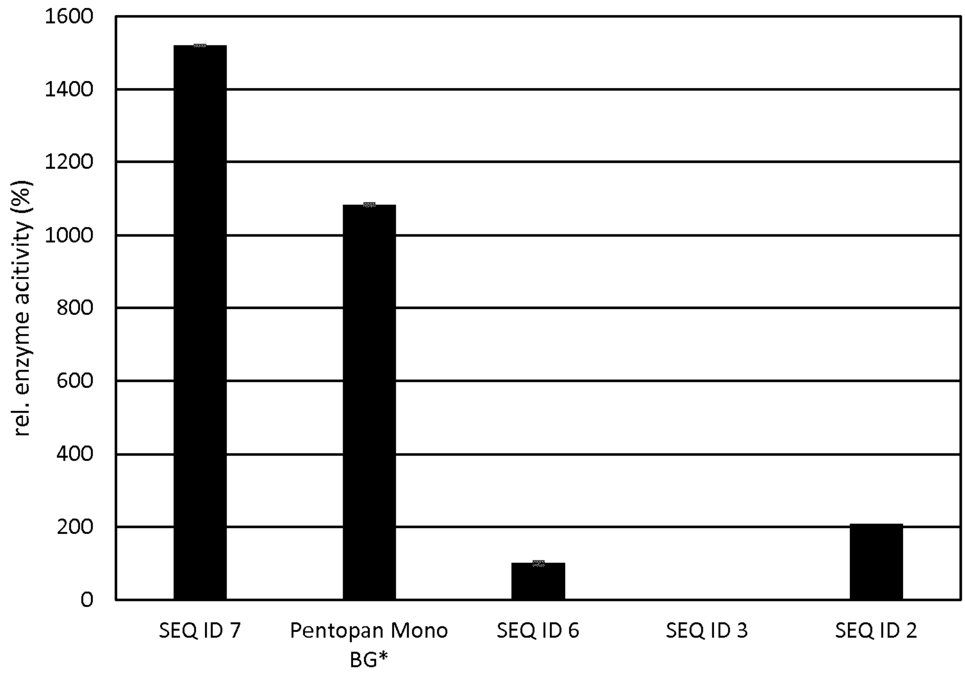
1600
1400
1200
1000
800
600
400
200
0
rel. enzyme acitivity (%)
SEQ ID 7
Pentopan Mono BG*
SEQ ID 6
SEQ ID 3
SEQ ID 2

**Fig. 4**

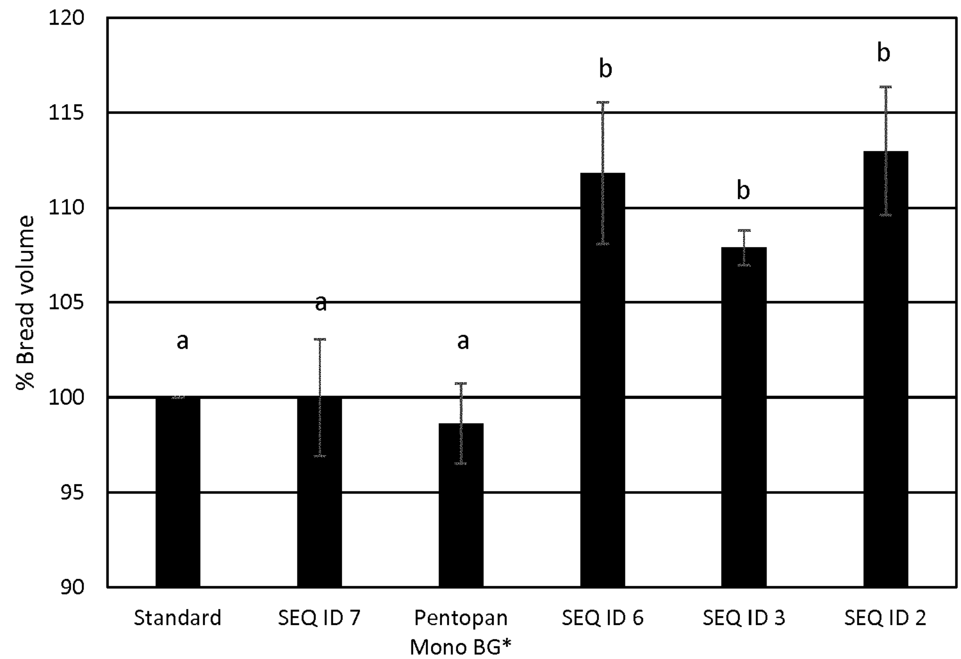
120
115
110
105
100
95
90
% Bread volume
a
a
a
b
b
b
Standard
SEQ ID 7
Pentopan Mono BG*
SEQ ID 6
SEQ ID 3
SEQ ID 2

**Fig. 5**

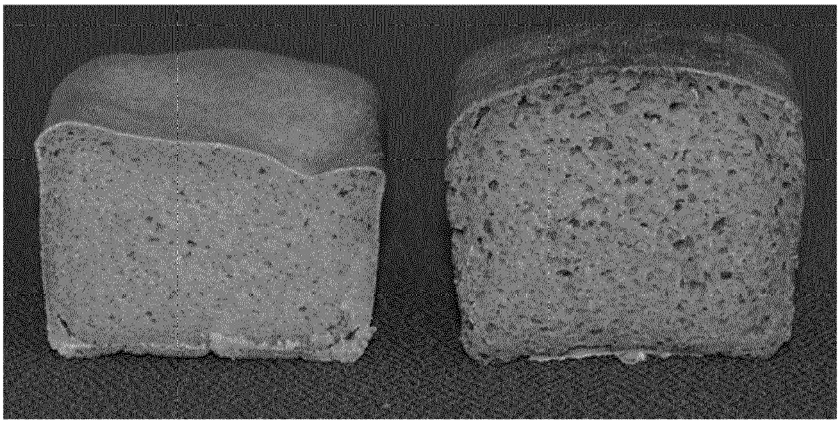

**Fig. 6**

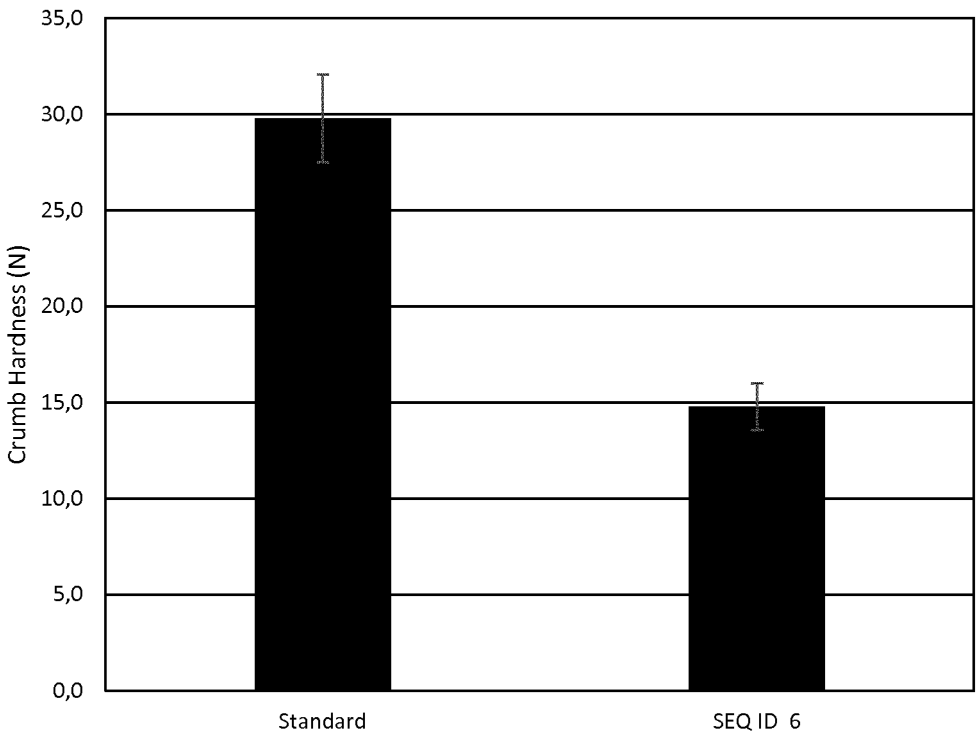
35,0
30,0
25,0
20,0
15,0
10,0
5,0
0,0
Crumb Hardness (N)
Standard
SEQ ID 6

Fig. 7

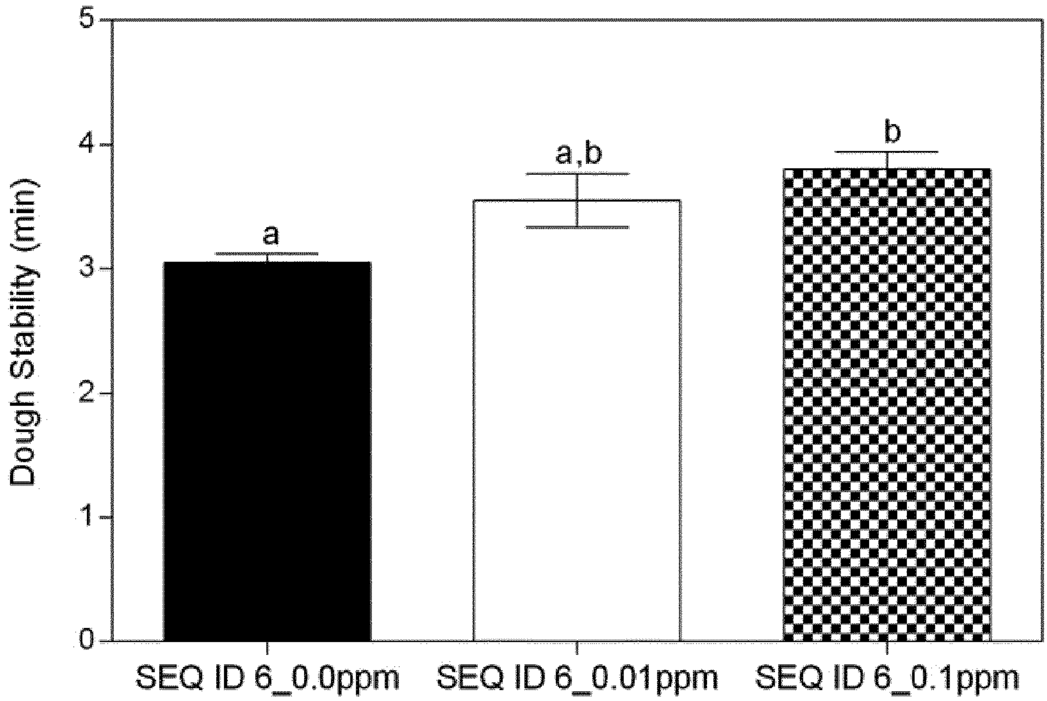
5
4
3
2
1
0
Dough Stability (min)
a
a,b
b
SEQ ID 6_0.0ppm
SEQ ID 6_0.01ppm
SEQ ID 6_0.1ppm

Fig. 8

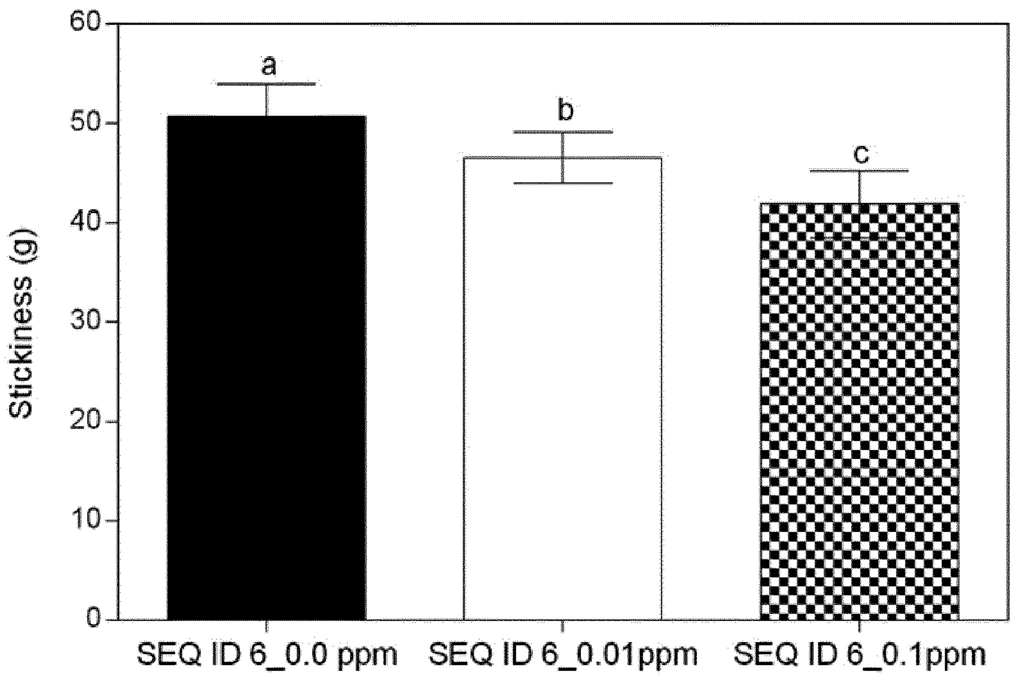
60
50
40
30
20
10
0
Stickiness (g)
a
b
c
SEQ ID 6_0.0 ppm
SEQ ID 6_0.01ppm
SEQ ID 6_0.1ppm

Fig. 9

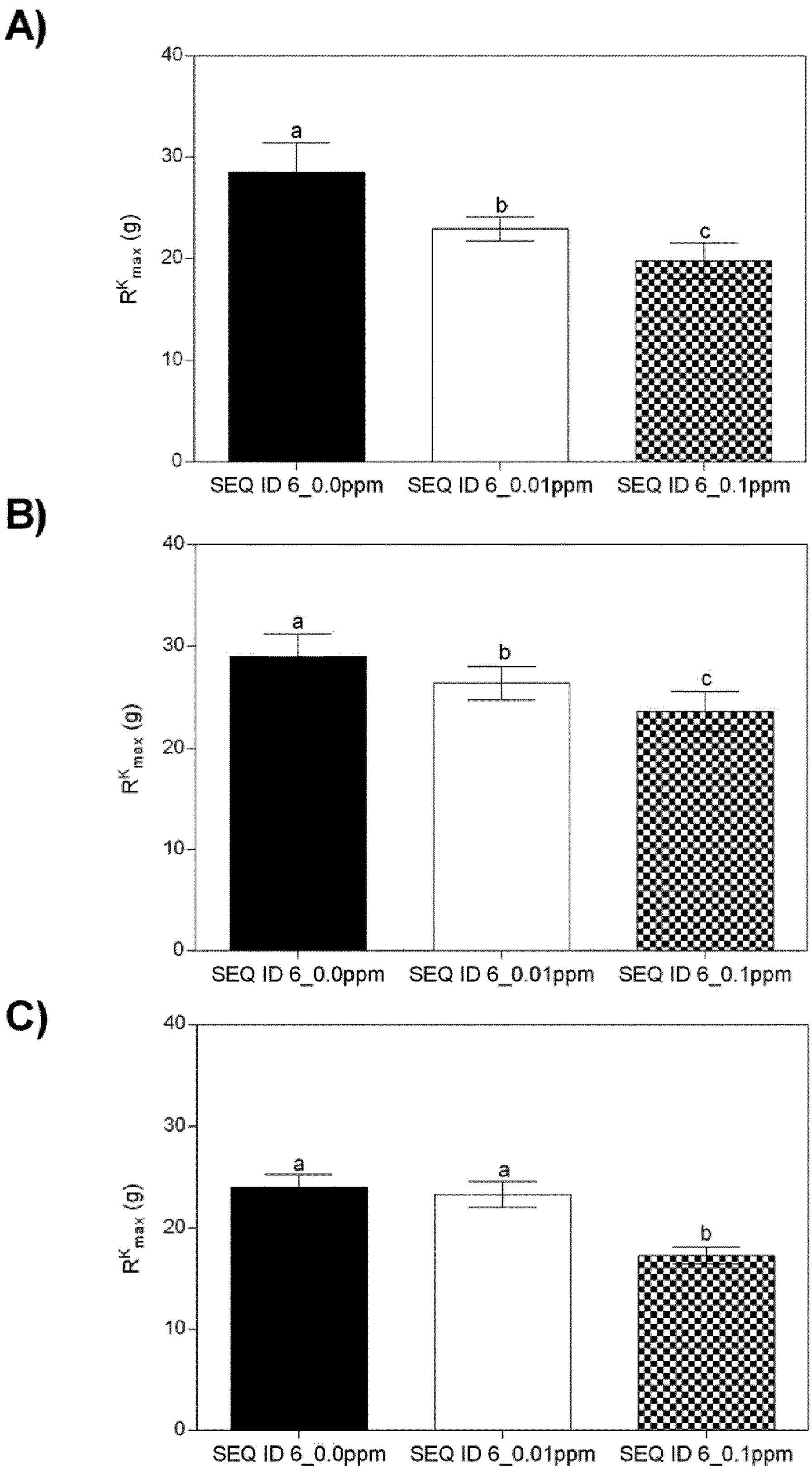
A)
B)
C)
40
30
20
10
0
$R^K_{max}$ (g)
a
b
c
a
SEQ ID 6_0.0ppm
SEQ ID 6_0.01ppm
SEQ ID 6_0.1ppm

Europäisches Patentamt
European Patent Office
Office européen des brevets

# EUROPEAN SEARCH REPORT

Application Number
EP 17 20 2797

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | WO 2009/146464 A2 (EDENSPACE SYSTEMS CORP [US]; PAPPAN KIRK [US]; NAIR RAMESH [US]; CHUML) 3 December 2009 (2009-12-03)<br>* sequence 62 *<br>* paragraphs [0054], [0209]; claims 1,9,14,47,51 *<br>----- | 12-15<br><br><br>11 | INV.<br>A21D2/26<br>C12N9/24 |
| X | DATABASE WPI<br>Week 201258<br>Thomson Scientific, London, GB;<br>AN 2012-L23745<br>XP002777307,<br>-& TW 201 122 105 A (ACAD SINICA)<br>1 July 2011 (2011-07-01)<br>* abstract *<br>* example 6; sequence 3 *<br>----- | 13-15 | |
| X<br><br><br>Y | US 2010/040736 A1 (VAN HAESENDONCK INGRID PAULA HILDE [BE] ET AL)<br>18 February 2010 (2010-02-18)<br>* paragraphs [0112], [0115] *<br>* paragraphs [0160] - [0162] *<br>* paragraphs [0196] - [0201] *<br>* tables 6,7 *<br>----- | 1-10<br><br><br>11 | TECHNICAL FIELDS SEARCHED (IPC)<br>A21D<br>C12N |

The present search report has been drawn up for all claims

1

| Place of search | Date of completion of the search | Examiner |
|---|---|---|
| Munich | 16 January 2018 | Götz, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 17 20 2797

This annex lists the patent family members relating to the patent documents cited in the above-mentioned European search report. The members are as contained in the European Patent Office EDP file on
The European Patent Office is in no way liable for these particulars which are merely given for the purpose of information.

16-01-2018

| Patent document cited in search report | Publication date | Patent family member(s) | Publication date |
|---|---|---|---|
| WO 2009146464 A2 | 03-12-2009 | US 2010017916 A1 | 21-01-2010 |
| | | WO 2009146464 A2 | 03-12-2009 |
| TW 201122105 A | 01-07-2011 | NONE | |
| US 2010040736 A1 | 18-02-2010 | AU 2008207001 A1 | 24-07-2008 |
| | | BR PI0806219 A2 | 30-08-2011 |
| | | CA 2671439 A1 | 24-07-2008 |
| | | CN 101646350 A | 10-02-2010 |
| | | EP 2106215 A2 | 07-10-2009 |
| | | RU 2009126835 A | 27-02-2011 |
| | | US 2010040736 A1 | 18-02-2010 |
| | | WO 2008087167 A2 | 24-07-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0056900 A
- WO 9600787 A
- WO 9533836 A
- WO 0024883 A
- WO 2014019219 A1


### Non-patent literature cited in the description


- Useful proteins from recombinant bacteria. *Scientific American,* 1980, vol. 242, 74-94
- Protein Purification. VCH Publishers, 1989
- Method 74-10.02. Measurement of Bread Firmness-Compression Test. AACC International, 2011; AACC International. Approved methods of analysis. AACC International, 06 January 2011
- Method 54-21.02. Measurement of Bread Firmness-Compression Test. AACC International, 2011; AACC International. Approved methods of analysis. AACC International, 06 January 2011
- **ADELSBERGER, H. ; HERTEL, C. ; GLAWISCHNIG, E. ; ZVERLOV, V.V. ; SCHWARZ, W.H.** Enzyme system of Clostridium stercorarium for hydrolysis of arabinoxylan: reconstitution of the in vivo system from recombinant enzymes. *Microbiology,* 2004, vol. 150 (7), 2257-2266
- **BIELY, P. ; SINGH, S. ; PUCHART, V.** Towards enzymatic breakdown of complex plant xylan structures: state of the art. *Biotechnol. Adv.,* 2016, vol. 34 (7), 1260-1274
- **BIELY, P. ; VRSANSKÁ, M. ; TENKANEN, M. ; KLUEPFEL, D.** Endo-beta-1,4-xylanase families: differences in catalytic properties. *Journal of biotechnology,* 1997, vol. 57 (1-3), 151-166
- **BOURAOUI, H. ; DESROUSSEAUX, M.-L. ; IOANNOU, E. ; ALVIRA, P. ; MANAI, M. ; REMOND, C. ; DUMON, C. ; FERNANDEZ-FUENTES, N. ; O'DONOHUE, M.J.** The GH51 alpha-L-arabinofuranosidase from Paenibacillus sp. THS1 is multifunctional, hydrolyzing main-chain and side-chain glycosidic bonds in heteroxylans. *Biotechnology for biofuels,* 2016, vol. 9, 140
- **BROCKMEIER, U. ; CASPERS, M. ; FREUDL, R. ; JOCKWER A. ; NOLL, T. ; EGGERT T.** Systematic Screening of All Signal Peptides from Bacillus subtilis: A Powerful Strategy in Optimizing Heterologous Protein Secretion in Gram-positive Bacteria. *Journal of Molecular Biology,* 2006, vol. 362 (3), 393-402
- **CHEN, W. Z. ; HOSENEY, R.C.** Development of an Objective Method for Dough Stickiness. *LWT - Food Science and Technology,* 1995, vol. 28 (5), 467-473
- **COLLINS, T. ; GERDAY, C. ; FELLER, G.** Xylanases, xylanase families and extremphilic xylanases. *FEMS Microbiology Reviews,* 2005, vol. 29, 3-23
- **COURTIN, C. M. ; DELCOUR, J. A.** Arabinoxylans and Endoxylanases in Wheat Flour Bread-making. *Journal of Cereal Science,* 2002, vol. 35, 225-243
- **COURTIN, C. M. ; ROELANTS, A. ; DELCOUR, J. A.** Reconstitution Experiments Provide Insight into the Role of Endoxylanases in Bread-Making. *Journal of Agricultural and Food Chemistry,* 1999, vol. 47 (5), 1870-1877
- **DE BOER, H. A. ; COMSTOCK, L. J. ; VASSER, M.** The tac promoter: a functional hybrid derived from the trp and lac promoters. *Proc. Natl Aca. Sci. USA,* 1983, vol. 80, 21-25
- **DÖRING, C. ; HUSSEIN, M. A. ; JEKLE, M. ; BECKER, T.** On the assessments of arabinoxylan localization and enzymatic modifications for enhanced protein networking and its structural impact on rye dough and bread. *In Food Chemistry,* 2017, vol. 229, 178-187
- **DÖRING C. ; GROSSMANN, I. ; ROTH, M. ; JEKLE, M. ; KOEHLER, P. ; BECKER, T.** Effect of rye bran particles on structure formation properties of rye dough and bread. Food Processing and Preservation. *Journal of Food Processing and Preservation,* 2017, vol. 41 (4
- **EVAN, G. I. ; LEWIS, G. K. ; RAMSAY, G. ; BISHOP, J.M.** Isolation of monoclonal antibodies specific for human c-myc proto-oncogene product. *Mol Cell Biol.,* December 1985, vol. 5 (12), 3610-6
- **GEMS, D. ; JOHNSTONE, I. L. ; CLUTTERBUCK, A. J.** An autonomously replicating plasmid transforms Aspergillus nidulans at high frequency. *Gene,* 01 February 1991, vol. 98 (1), 61-7

- **GUO ; SHERMAN.** *Molecular Cellular Biology,* 1995, vol. 15, 5983-5990
- **IZYDORCZYK, M. S. ; BILIADERIS, C. G.** Cereal arabinoxylans: advances in structure and physicochemical properties. *Carbohydr. Polym.,* 1995, vol. 28, 33-48
- **JOHNSON, E.A. ; SAKAJOH, M. ; HALLIWELL, G. ; MADIA, A. ; DEMAIN, A. L.** Saccharification of Complex Cellulosic Substrates by the Cellulase System from Clostridium thermocellum. *Appl. Environ. Microbiol.,* 1982, vol. 43 (5), 1125-1132
- **KNUDSEN, K. E. B.** Fiber and nonstarch polysaccharide content and variation in common crops used in broiler diets. *Poultry Science,* 2014, vol. 93, 2380-2393
- **KOECK, D.E. ; MECHELKE, M. ; ZVERLOV, V.V. ; LIEBL, W. ; SCHWARZ, W.H.** Herbivorax saccincola gen. nov., sp. nov., a cellulolytic, anaerobic, thermophilic bacterium isolated via in sacco enrichments from a lab scale biogas reactor. *International Journal of Systematic and Evolutionary Microbiolog,* 2016, vol. 66, 4458-4463
- **KOLENOVÁ, K. ; VRŠANSKÁ, M. ; BIELY, P.** Mode of endo-β, 4-xylanases of families 10 and 11 on acidic xylooligosaccharides. *Journal of Biotechnology,* 2006, vol. 121, 338-345
- **KORZ, D. J. ; RINAS, U. ; HELLMUTH K. ; SANDERS, E. A. ; DECKWER, W.D.** Simple fed-batch technique for high cell density cultivation of Escherichia coli. *J. Biotechnol.,* 1995, vol. 39, 59-65
- **KROLL, J. ; STEINLE, A. ; REICHELT, R. ; EWERING, C. ; STEINBÜCHEL, A.** Establishment of a novel anabolism-based addiction system with an artificially introduced mevalonate pathway: Complete stabilization of plasmids as universal application in white biotechnology. *Metabolic Engineering,* 2009, vol. 11, 168-177
- **LARSON, S. B. ; DAY, J. ; BARBA DE LA ROSA, A. P. ; KEEN, N. T. ; MCPHERSON, A.** First crystallographic structure of a xylanase from glycoside hydrolase family 5: implications for catalysis. *Biochemistry,* 2003, vol. 42, 8411-8422
- **LIN-CEREGHINO, J. ; WON, W. W. ; XIONG, S. ; GIAN, W. ; LUONG, L.T. ; VU, J. ; JOHNSON, S.D. ; LIN-CEREGHINO G.P.** Condensed protocol for competent cell preparation and transformation of the methylotrophic yeast Pichia pastoris. *Biotechniques,* 2005, vol. 38 (1), 44, , 46, , 48
- **LOMBARD V. ; GOLACONDA R.H. ; DRULA E. ; COUTINHO P.M. ; HENRISSAT B.** The Carbohydrate-active enzymes database (CAZy) in 2013. *Nucleic Acids Res,* 2014, vol. 42, D490-D495
- **MEUSER, F. ; SUCKOW, P.** Non-starch polysaccharides. In Chemistry and Physics of Baking. The Royal Society of Chemistry, 1986, 42-61
- **OLOFFS K ; JEROCH H ; SCHÖNER FJ.** Efficacy of non-starch polysaccharide hydrolyzing enzymes on nutrient digestibility and gross energy convertibility of barley-rye and wheat-rye based diets for laying hens. *Arch_Tierernahr.,* 1999, vol. 52 (2), 155-65
- **PARK, Y.S. ; KAI, K. ; LIJIMA, S. ; KOBAYASHI, T.** Enhanced β-Galactosidase Production by High Cell-Density Culture of Recombinant Bacillus subtilis with Glucose Concentration control. *Biotechnol Bioeng.,* 1991, vol. 40 (6), 686-696
- **ROMANOS, M. A. ; SCORER, C. A. ; JEFFREY, J. C.** Foreign Gene Expression in Yeast: a Review. *YEAST,* 1992, vol. 8, 423-488
- **SAMBROOK, J ; RUSSELL, D. W.** Molecular cloning: a laboratory manual. Coldspring-Harbour Laboratory Press, 2001
- **SAMBROOK, J. ; FRITSCH, E. F. ; MANIATIS. T.** Molecular cloning: a laboratory manual. Cold Spring Harbor Laboratory Press, 1989
- **SIMONEN, M. ; PALVA, I.** Protein secretion in Bacillus species. *Microbiological Reviews,* 1993, vol. 57, 109-137
- **VILLA-KOMAROFF, L. ; EFSTRATIADIS, A. ; BROOME, S. ; LOMEDICO, P. ; TIZARD, R. ; NABER, S. P. ; CHICK, W. L. ; GILBERT, W.** A bacterial clone synthesizing proinsulin. *Proc. Natl. Acad. Sci. USA,* 1978, vol. 75, 3727-3731
- **WENCHE FRØLICH, W. ; AMAN, P. ; INGE TETENS, T.** Whole grain foods and health a Scandinavian perspective. *Food & Nutrition Research,* 2013, 57
- **WILSON, I. A. ; NIMAN, H. L. ; HOUGHTEN, R. A. ; CHERENSON, A. R. ; CONOLLY, M. L. ; LERNER, R. A.** The structure of an antigenic determinant in a protein. *Cell,* 1984, vol. 37 (3), 767-78
- Methods for measuring cellulase activities. **WOOD, T. M. ; BHAT, K. M.** Methods in Enzymology. Academic Press, 1988, vol. 160, 87-112
- **YIN, Y.B. ; MAO, X.Z. ; YANG, J.C. ; CHEN, X. ; MAO, F.L. ; XU, Y.** dbCAN: a web resource for automated carbohydrate-active enzyme annotation. *Nucleic Acids Res,* 2012, vol. 40, W445-W451